(19) Europäisches Patentamt
European Patent Office
Office européen des brevets

(11) Veröffentlichungsnummer: **0 299 389 A2**

# (12) EUROPÄISCHE PATENTANMELDUNG

(21) Anmeldenummer: **88110991.2**

(22) Anmeldetag: **09.07.88**

(51) Int. Cl.4: **C07D 495/04 , A61K 31/505 , //(C07D498/04,333:00,239:00)**

Patentansprüche für folgende Vertragsstaaten: ES + GR.

(30) Priorität: **15.07.87 DE 3723327**

(43) Veröffentlichungstag der Anmeldung: **18.01.89 Patentblatt 89/03**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Nimmesgern, Hildegard, Dr.**
**Rauenthaler Weg 32**
**D-6000 Frankfurt am Main 71(DE)**
Erfinder: **Weidmann, Klaus, Dr.**
**Talweg 11**
**D-6242 Kronberg/Taunus(DE)**
Erfinder: **Lang, Hans-Jochen, Dr.**
**Rüdesheimer Strasse 7**
**D-6238 Hofheim am Taunus(DE)**
Erfinder: **Rippel, Robert, Dr.**
**Frankfurter Strasse 66**
**D-6238 Hofheim am Taunus(DE)**
Erfinder: **Herling, Andreas W., Dr.**
**Am Walberstück 5**
**D-6277 Bad Camberg(DE)**

(54) **Substituierte Thienoimidazol-Derivate, Verfahren zu ihrer Herstellung, sie enthaltende pharmazeutische Zubereitungen und ihre Verwendung als Magensekretionshemmer, Magenschutzmittel sowie als Medikament gegen intestinale Entzündungen.**

(57) Die Erfindung betrifft Verbindungen der Formel

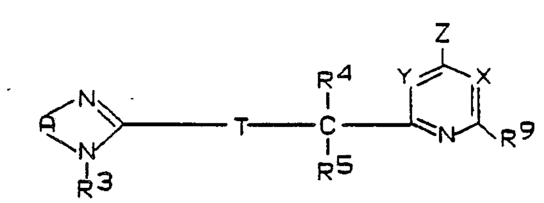

(I)

in welcher

A für a) 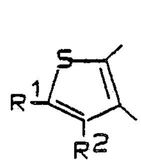 , b) 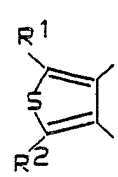 oder c) 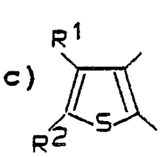 steht,

T -S-, -SO- oder $-SO_2-$ bedeutet,
$R^1$ bis $R^{10}$, X, Y, Z, die in der Beschreibung angegebenen Bedeutungen haben, Verfahren zu ihrer

EP 0 299 389 A2

Herstellung, sie enthaltende pharmazeutische Zubereitungen und ihre Verwendung als Magensäuresekretionshemmer, Magenschutzmittel sowie als Medikament gegen intestinale Entzündungen.

**Substituierte Thienoimidazol-Derivate, Verfahren zu ihrer Herstellung, sie enthaltende pharmazeutische Zubereitungen und ihre Verwendung als Magensäuresekretionshemmer, Magenschutzmittel sowie als Medikament gegen intestinale Entzündungen**

Benzimidazol-Derivate mit magensäuresekretionshemmender Wirkung sind z.B. aus DE-A-25 48 340, EP-A-5129, EP-A-178 438, EP-A-198 583 und DE-A-32 40 248 bekannt.

Die vorliegende Erfindung betrifft Thienoimidazol-Derivate der Formel I

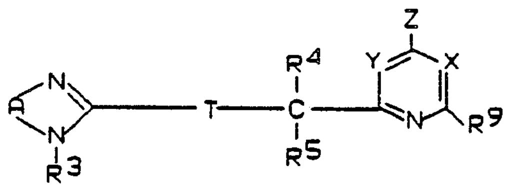

(I)

in welcher

A für a) 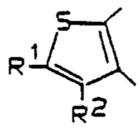 , b) 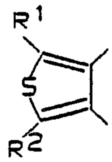 oder c) 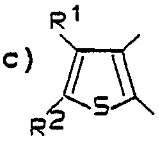 steht,

T -S-, -SO- oder $-SO_2-$ bedeutet,

$R^1$ und $R^2$ gleich oder verschieden sind und Wasserstoff, Halogen, Cyano, Nitro, Trifluormethyl, $(C_1$-$C_6)$-Alkyl, $(C_1$-$C_6)$-Hydroxyalkyl, $(C_1$-$C_6)$-Alkoxy, $(C_1$-$C_4)$-Fluoralkoxy, $-OCF_2Cl$, $-O-CF_2-CHFCl$, $(C_1$-$C_6)$-Alkylmercapto, $(C_1$-$C_6)$-Alkylsulfinyl, $(C_1$-$C_6)$-Alkylsulfonyl, $(C_1$-$C_6)$-Alkylcarbonyl, $(C_1$-$C_6)$-Alkoxycarbonyl, Carbamoyl, N-$(C_1$-$C_4)$-Alkylcarbamoyl, N,N-Di-$(C_1$-$C_4)$-alkylcarbamoyl, $(C_1$-$C_6)$-Alkylcarbonyloxy, $(C_3$-$C_8)$-Cycloalkyl, Phenyl, Benzyl, Phenoxy, Benzyloxy, Anilino, N-Methylanilino, Phenylmercapto, Phenylsulfonyl, Phenylsulfinyl, Sulfamoyl, N-$(C_1$-$C_4)$-Alkylsulfamoyl oder N,N-Di-$(C_1$-$C_4)$-alkylsulfamoyl bedeuten, oder, falls A wie oben unter (a) oder (c) definiert ist, auch gemeinsam $-[CH_2]_n-$ oder -CH=CH-CH=CH- bedeuten können, wobei eine $CH_2$-Gruppe gegebenenfalls durch O, S, SO oder $SO_2$ ersetzt ist,

$R^3$ Wasserstoff, Alkanoyl, $(C_1$-$C_6)$-Alkylcarbamoyl oder eine andere physiologisch verträgliche, vorzugsweise im sauren Medium und/oder unter physiologischen Bedingungen abspaltbare $N^{im}$-Schutzgruppe bedeutet,

$R^4$ und $R^5$ gleich oder verschieden sind und Wasserstoff oder $(C_1$-$C_3)$-Alkyl bedeuten,

X Stickstoff bedeutet und Y $CR^6$ bedeutet, oder

X $CR^6$ bedeutet und Y Stickstoff bedeutet,

$R^6$ Wasserstoff, Halogen, $(C_1$-$C_6)$-Alkyl, Trifluormethyl, $(C_1$-$C_6)$-Alkoxy, $-O-C_pH_{(2p+1-q)}F_q$, $(C_1$-$C_6)$-Alkoxy-$(C_1$-$C_6)$-alkyl, $(C_6$-$C_{12})$-Aryl, $(C_6$-$C_{12})$-Aryl-$(C_1$-$C_6)$-alkoxy, $(C_1$-$C_9)$-Heteroaryl, $(C_6$-$C_{12})$-Aryloxy, wobei der entsprechende Arylteil gegebenenfalls durch Halogen oder Trifluormethyl substituiert sein kann, Cyano, Nitro, $(C_1$-$C_6)$-Alkylamino, Di-$(C_1$-$C_6)$-alkylamino oder Amino bedeutet,

Z $NR^7R^8$, $OR^{10}$ oder $SR^{10}$ bedeutet,

$R^7$ und $R^8$ gleich oder verschieden sind und Wasserstoff, $(C_1$-$C_6)$-Alkyl, $(C_6$-$C_{12})$-Aryl, $(C_7$-$C_{13})$-Aralkyl oder $(C_3$-$C_6)$-Cycloalkyl bedeuten oder zusammen mit dem Stickstoff, an den die gebunden sind, für Azetidino, Pyrrolidino, Piperidino, Piperazino, Morpholino oder N-$(C_1$-$C_4)$-Alkylpiperazino stehen, die gegebenenfalls durch eine oder zwei gleiche oder verschiedene $(C_1$-$C_6)$-Alkylgruppen substituiert sind,

$R^9$ Wasserstoff, Halogen, $(C_1$-$C_3)$-Alkyl, $(C_1$-$C_4)$-Alkoxy, Benzyloxy oder $(C_1$-$C_7)$-Alkoxy-$(C_1$-$C_3)$-alkyl bedeutet,

$R^{10}$ Wasserstoff, $(C_1$-$C_4)$-Alkyl, $(C_7$-$C_{13})$-Aralkyl, $(C_6$-$C_{12})$-Aryl, Vinyl, Allyl oder einen substituierten Alkylrest der Formel $C_pH_{(2p+1-q)}F_q$ bedeutet,

n = 3 oder 4 ist,

p = 1, 2, 3 oder 4 ist, insbesondere 2 oder 4, und

q = 1 bis (2 p + 1) ist, insbesondere 3 bis 7,

sowie deren physiologisch verträglichen Salze.

1H-Thieno[3,4-d]imidazol-Derivate der Formel I, worin A wie oben unter (b) definiert ist, sind bevorzugt.

Weiterhin sind Verbindungen der Formel I bevorzugt, in welchen $R^9$ für Wasserstoff steht. T ist vorzugsweise eine -SO-Gruppe.

Besonders bevorzugt sind Verbindungen der Formel I, worin

A vorzugsweise wie oben unter (b) definiert ist,

T vorzugsweise eine -SO-Gruppe bedeutet,

$R^1$ und $R^2$ gleich oder verschieden sind und Wasserstoff, ($C_1$-$C_3$)-Alkyl, Halogen, ($C_1$ $C_4$)-Alkoxy oder ($C_1$-$C_4$)-Alkoxycarbonyl bedeuten,

$R^3$ wie oben definiert ist,

$R^4$ und $R^5$ jeweils Wasserstoff bedeuten,

$R^6$ Amino, Wasserstoff, Halogen, ($C_1$-$C_6$)-Alkyl, ($C_1$-$C_6$)-Alkoxy, -O-$C_pH_{(2p+1-q)}F_q$, ($C_6$-$C_{12}$)-Aryloxy, ($C_6$-$C_{12}$)-Aryl-($C_1$-$C_6$)-alkoxy, wobei der entsprechende Arylteil gegebenenfalls durch Halogen oder Trifluormethyl substituiert sein kann, Cyano, Nitro, ($C_1$-$C_6$)-Alkylamino, Di-($C_1$-$C_6$)-alkylamino oder ($C_1$-$C_6$)-Alkoxy-($C_1$-$C_3$)-alkyl bedeutet, wobei $R^6$ insbesondere für Wasserstoff, Chlor, ($C_1$-$C_6$)-Alkyl, ($C_1$-$C_6$)-Alkoxy oder $C_pH_{(2p+1-q)}F_q$ steht,

$R^7$ und $R^8$ zusammen mit dem Stickstoff, an das sie gebunden sind, für Azetidino, Piperazino, Pyrrolidino, Morpholino, Piperidino oder N-($C_1$-$C_4$)-Alkylpiperazino stehen oder beide Wasserstoff bedeuten oder $R^7$ Wasserstoff und $R^8$ eine ($C_3$-$C_6$)-Cycloalkyl-Gruppe sind, bevorzugt aber gleich oder verschieden sind, und für Wasserstoff oder ($C_1$-$C_6$)-Alkyl oder gemeinsam für Piperidino, Pyrrolidino, Piperazino oder Morpholino stehen und/oder

$R^9$ Wasserstoff ist,

$R^{10}$ Wasserstoff, ($C_1$-$C_4$)-Alkyl, ($C_6$-$C_{12}$)-Aryl, ($C_7$-$C_{13}$)-Aralkyl, Vinyl, Allyl, $C_pH_{(2p+1-q)}F_q$ bedeutet, wobei Alkyl bevorzugt Methyl, Ethyl, Isopropyl, n-Propyl, n-Butyl, sec-Butyl oder tert-Butyl ist und Aralkyl bevorzugt Benzyl ist,

insbesondere aber Verbindungen der Formel I, worin

A vorzugsweise wie oben unter (b) definiert ist,

T vorzugsweise eine -SO-Gruppe bedeutet,

$R^1$ und $R^2$ gleich oder verschieden sind und Wasserstoff oder ($C_1$-$C_3$)-Alkyl bedeuten,

$R^3$ Wasserstoff bedeutet,

$R^4$ und $R^5$ jeweils Wasserstoff bedeuten,

$R^6$ Wasserstoff, ($C_1$-$C_6$)-Alkoxy, Trifluorethoxy, Heptafluorbutoxy, Benzyloxy, Fluorbenzyloxy, Difluorbenzyloxy, Trifluorbenzyloxy, Amino, Ethyl oder Methyl, Nitro, Cyano oder Chlor bedeutet,

$R^9$ Wasserstoff bedeutet,

$R^7$ und $R^8$ gleich sind und zusammen mit dem Stickstoff, an den sie gebunden sind, einen Pyrrolidin-, Morpholin-, Piperidin oder Piperazin-Ring bilden oder Wasserstoff, Methyl oder Ethyl bedeuten.

Alkylgruppen alleine oder als Teil einer anderen Gruppe (z.B. Alkoxy, Alkoxycarbonyl, Alkylmercapto, Alkylsulfinyl, Alkylsulfonyl, Aralkyl oder Alkanoyl) sind geradkettig oder verzweigt und sind z.B. Methyl, Ethyl, n-Propyl, Isopropyl, Isobutyl, sec-Butyl, n-Butyl, n-Pentyl, Isopentyl oder n-Hexyl.

($C_1$-$C_6$)-Alkoxygruppen sind bevorzugt Methoxy oder Ethoxy. Bevorzugte ($C_1$-$C_6$)-Alkoxycarbonylgruppen sind Methoxycarbonyl oder Ethoxycarbonyl. Unter ($C_6$-$C_{12}$)-Aryl-($C_1$-$C_6$)-alkoxy versteht man bevorzugt Benzyloxy.

Bevorzugte ($C_1$-$C_6$)-Alkanoylgruppen sind Formyl oder Acetyl.

($C_3$-$C_8$)-Cycloalkyl-Gruppen sind beispielsweise Cyclopropyl, Cyclobutyl, Cyclopentyl und Cyclohexyl, bevorzugte ($C_3$-$C_6$)-Cycloalkyl-Gruppen sind Cyclopentyl oder Cyclohexyl.

Aryl ist beispielsweise Phenyl, Naphthyl oder p-Biphenylyl, bevorzugt ist Phenyl. Entsprechendes gilt für davon abgeleitete Reste, wie Aralkyl, Aryloxy, Aralkyloxy oder Aroyl. Unter ($C_1$-$C_9$)-Heteroaryl werden Reste 5- oder 6-gliedriger monocyclischer oder 9- oder 10-gliedriger bicyclischer Heteroaromaten mit mindestens 1 C-Atom, 1 bis 4 N-Atomen und/oder 1 S- oder O-Atom als Ringglieder verstanden, wie sie beispielsweise in Katritzky, Lagowski, Chemie der Heterocyclen, Berlin, Heidelberg 1968, Seite 3 bis 5 definiert sind. Monocyclische Heteroaromaten sind z.B. Thiophen, Furan, Pyrrol, Imidazol, Pyrazol, Pyridin, Tetrazol, Pyrazin, Pyrimidin, Pyridazin, 1,2,4-Triazol, Thiazol, Isothiazol, Oxazol und Isoxazol. Bicyclische Heteroaromaten sind z.B. Benzothiophen, Benzofuran, Indol, Isoindol, Indazol, Benzimidazol, Chinolin, Isochinolin, Phthalazin, Chinoxalin, Chinazolin und Cinnolin.

Halogen ist Fluor, Chlor, Brom oder Jod. Bevorzugt sind Fluor und Chlor.

Als Salze kommen insbesondere Salze mit physiologisch verträglichen Säuren und Alkali- und Erdalkalisalze und Salze mit physiologisch verträglichen Aminen in Frage, die z.B. durch Umsetzung mit HCl, $H_2SO_4$, Phosphorsäure, alicyclischen, aromatischen oder heteroaromatischen Carbon- oder Sulfonsäuren, mit Methionin, Tryptophan, Lysin, Arginin oder ähnlichem gebildet werden.

$R^3$ steht vorzugsweise für Wasserstoff, ($C_1$-$C_6$)-Alkylcarbamoyl oder einen Rest der Formel VI,

$-(CO-O-)_p\ (CR^{11}R^{12}-O-)_q$ W-B (VI)

worin p = 0 oder 1, q = 0 oder 1, W eine Bindung oder -CO-, $-CR^{13}R^{14}$-oder $-CO-CR^{13}R^{14}$- und B Wasserstoff, einen Acylrest oder einen gegebenenfalls substituierten Alkylrest bedeuten.

$R^{11}$ und $R^{12}$ sind gleich oder verschieden und bedeuten Wasserstoff, $(C_1-C_6)$-Alkyl, $(C_3-C_8)$-Cycloalkyl, $(C_7-C_{11})$-Aralkyl oder $(C_6-C_{12})$-Aryl.

B und $R^{11}$ können auch gemeinsam für eine $-[CH_2]_r$-Kette mit r = 3, 4 oder 5 - vorzugsweise 4 - stehen, wobei an einer oder mehreren der $CH_2$-Gruppen jeweils ein Wasserstoffatom durch OH, geschütztes OH, Amino, Acylamino und/oder Halogen ersetzt sein kann. Bei einem Rest mit substituierter $-[CH_2]_r$-Kette handelt es sich vorzugsweise um einen gegebenenfalls mit in der Kohlenhydratchemie üblichen Schutzgruppen teilweise oder vollständig geschützten Glycosylrest, der sich von einer Glycopyranose, Glycofuranose oder einem Oligosaccharid ableitet.

Der Glycosylrest kann sowohl α- als auch β-glycosidisch verknüpft sein.

Er kann beispielsweise einen Glycofuranosyl- oder Glycopyranosyl-Rest sein, der sich von natürlich vorkommenden Aldotetrosen, Aldopentosen, Aldohexosen, Ketopentosen, Desoxyaldosen, Aminoaldosen und Oligosacchariden, wie Di- und Trisacchariden, sowie deren Stereoisomeren ableiten.

Diese Glycosylreste leiten sich insbesondere von natürlichen, in Mikroorganismen, Pflanzen, Tieren oder Menschen vorkommenden D- oder L-Monosacchariden wie Ribose (Rib), Arabinose (Ara), Xylose (Xyl), Lyxose (Lyx), Allose (All), Altrose (Alt), Glucose (Glc), Mannose (Man), Gulose (Gul), Idose (Ido), Galactose (Gal), Talose (Tal), Erythrose (Ery), Threose (Thr), Psicose (Psi), Fructose (Fru), Sorbose (Sor), Tagatose (Tag), Xylulose (Xyu), Fucose (Fuc), Rhamnose (Rha), Olivose (Oli), Oliose (Olo), Mycarose (Myc), Rhodosamin (RN), N-Acetyl-glucosamin (GlcNAc), N-Acetyl-galactosamin (GalNAc), N-Acetyl-mannosamin (ManNAc) oder Disacchariden, wie Maltose (Mal), Lactose (Lac), Cellobiose (Cel), Gentibiose (Gen), N-Acetyl-lactosamin (LacNAc), Chitobiose (Chit), β-Galactopyranosyl-(1→3)-N-acetylgalactosamin und β-Galactopyranosyl-(1→3)- oder - (1→4)-N-acetyl-glucosamin, sowie deren synthetischen Derivate, wie 2-Desoxy-, 2-Amino-, 2-Acetamido- oder 2-Halogeno-, bevorzugt Bromo- oder Iodo-Zucker ab.

Unter den in der Kohlenhydratchemie üblichen Schutzgruppen versteht man z.B. die $(C_1-C_{10})$-Acylschutzgruppen wie $(C_1-C_6)$-Alkanoyl (z.B. Acetyl-, Trichloracetyl-, Trifluoracetyl-), Benzoyl- oder p-Nitrobenzoyl-, sowie gegebenenfalls modifizierte Methyl-, Methyloxymethyl-, Benzyl-, Tetrahydropyranyl-, Benzyliden-, Isopropyliden- oder Trityl-Gruppen, wobei hier die Acylschutzgruppen, insbesondere die Acetyl- (Ac)-Gruppe, bevorzugt sind.

a) Falls p = q = 0 ist, haben die Reste vorzugsweise folgende Bedeutungen:

W ist eine Bindung oder bedeutet -CO-, $-CR^{13}R^{14}$- oder $-CO-CR^{13}R^{14}$-.

B bedeutet Wasserstoff (nur, falls W keine Bindung ist), $(C_1-C_{10})$-Alkyl; $(C_2-C_{12})$-Alkenyl; $(C_3-C_{12})$-Cycloalkyl; $(C_6-C_{12})$-Aryl, das gegebenenfalls durch 1, 2 oder 3 gleiche oder verschiedene Reste aus der Reihe $(C_1-C_4)$-Alkyl, Chlor, Brom, Fluor, Nitro, Trifluormethyl, $(C_1-C_4)$-Alkoxy und Hydroxy substituiert ist; $-(CH_2)_s-CH(NH_2)-R^{15}$ mit s = 1 - 9; den Acylrest einer Aminosäure oder $(C_1-C_6)$-Alkyl, das durch bis zu 4 gleiche oder verschiedene Reste aus der Reihe F, Cl oder Br substituiert ist.

$R^{13}$ und $R^{14}$ sind gleich oder verschieden und bedeuten Wasserstoff, $(C_1-C_6)$-Alkyl, $(C_1-C_6)$-Alkoxy, $(C_3-C_8)$-Cycloalkyl, $(C_7-C_{11})$-Aralkyl, $(C_6-C_{12})$-Aryl oder Pyridyl oder $R^{13}$ und $R^{14}$ stehen gemeinsam für $-[CH_2]_4$-, $-[CH_2]_5$- oder-$[CH_2]_6$-, worin 1 oder 2 $CH_2$-Gruppen durch 0 ersetzt sein können.

$R^{15}$ bedeutet Wasserstoff oder $(C_1-C_{10})$-Alkyl.

b) Falls q = 1 ist, sind W und B wie oben unter (a) definiert. Darüber hinaus kann W -CO-O- und $-CO-O-CR^{13}R^{14}$- bedeuten, wobei $R^{13}$ und $R^{14}$ die obengenannten Bedeutungen haben. B kann auch im Falle von W = Bindung für Wasserstoff stehen.

c) Falls p = 1 und q = 0 ist, steht W für eine Bindung oder bedeutet $-CR^{13}R^{14}$-, wobei $R^{13}$ und $R^{14}$ die Bedeutungen wie unter (a) haben. B ist definiert wie unter (a), kann aber nicht für den Acylrest einer Aminosäure stehen. -CO-O-W-B kann darüber hinaus für weitere $N^{im}$-Schutzgruppen vom Urethantyp stehen, die von der vorstehenden Definition nicht umfaßt werden (vgl. z.B. Hubbuch, Kontakte Merck 3/79 14-23; Büllesbach, Kontakte Merck 1/80 23-35).

Unter einem gegebenenfalls substituierten $(C_6-C_{12})$-Arylrest (siehe oben unter (a)) wird beispielsweise Phenyl, (o-, m-, p-)Tolyl, (o-, m-, p-)Ethylphenyl, 2-Ethyl-p-tolyl, 4-Ethyl-o-tolyl, 5-Ethyl-m-tolyl, (o-, m- oder p-)Propylphenyl, 2-Propyl-(m- oder p-)Tolyl, 4-Isopropyl-2,6-xylyl, 3-Propyl-4-ethylphenyl, (2,3,4-, 2,3,6-, oder 2,4,5-)Trimethylphenyl, (o-, m- oder p-)Fluorphenyl, (o-, m- oder p-Trifluormethyl)phenyl, 4-Fluor-2,5-xylyl, (2,4-, 2,5-, 2,6-, 3,4- oder 3,5-)Difluorphenyl, (o-, m- oder p-)Chlorphenyl, 2-Chlor-p-tolyl, (3-, 4-, 5- oder 6-)Chlorotolyl, 4-Chlor-2-propylphenyl, 2-Isopropyl-4-chlorphenyl, 4-Chlor-3,5-xylyl, (2,3-, 2,4-, 2,5-, 2,6- oder 3,5-)Dichlorphenyl, 4-Chlor-3-fluorphenyl, (3- oder 4-)Chlor 2-fluorphenyl, (o-, m- oder p-)Trifluormethylphenyl, (o-, m- oder p-)Ethoxyphenyl, (4- oder 5-)Chlor-2-methoxyphenyl, 2,4-Dichlor-(5- oder 6-)methylphenyl oder (o-, m- oder p-)Methoxyphenyl verstanden.

($C_1$-$C_{10}$)-Alkyl ist beispielsweise Methyl, Ethyl, Propyl, Butyl, Pentyl, Hexyl, Heptyl, Octyl, Nonyl, Decyl oder deren isomere Formen.

($C_3$-$C_{12}$)-Cycloalkyl schließt alkylsubstituiertes Cycloalkyl und bi- und mehrcyclische Systeme mit ein. Es wird darunter beispielsweise verstanden: Cyclopropyl, 2-Methylcyclopropyl, 2,2-Dimethylcyclopropyl, 2,3-Diethylcyclopropyl, 2-Butylcyclopropyl, Cyclobutyl, 2-Methylcyclobutyl, 3-Propylcyclobutyl, 2,3,4-Triethylcyclobutyl, Cyclopentyl, 2,2-Dimethylcyclopentyl, 2-Pentylcyclopentyl, 3-tert-Butylcyclopentyl, 2,2-Dimethylcyclohexyl, Cycloheptyl, Cyclononyl, Cyclodecyl, Norbornyl oder Adamantyl.

Unter einem Acylrest einer Aminosäure wird vorzugsweise der Rest einer α-Aminosäure, insbesondere aus der Reihe der natürlich vorkommenden α-Aminosäuren oder deren Antipoden verstanden, wie z.B. H-Gly-, H-Ala-, H-Val-, H-Leu-, H-Ile, H-Phe-, H-Lys-, H-Pro-, H-Trp-, H-Met-, H-Ser-, H-Thr-, H-Cys-, H-Tyr-, H-Asn-, H-Gln-, H-Asp-, H-Glu- , H-Arg-, H-Orn- oder die entsprechenden Reste in der D-Konfiguration.

Ohne daß der Erfindungsgegenstand darauf beschränkt wäre, seien im folgenden einige erfindungsgemäße Urethanschutzgruppen $R^3$ = -CO-O-WB genannt: ($C_1$-$C_6$)-Alkoxycarbonyl wie Boc; ($C_3$-$C_{12}$)-Cycloalkyloxycarbonyl wie Mboc, Iboc oder Adoc;

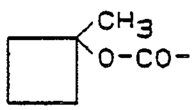

Mboc

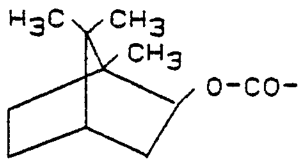

Iboc

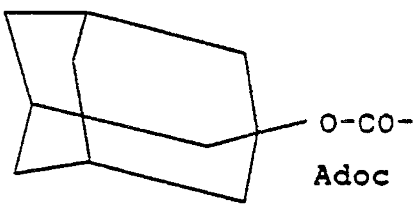

Adoc

($C_3$-$C_{12}$)-Cycloalkyl-($C_1$-$C_6$)-alkoxycarbonyl wie Adpoc;

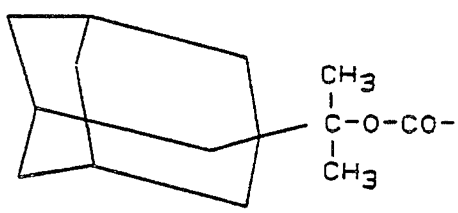

Adpoc

($C_6$-$C_{12}$)-Aryl-($C_1$-$C_6$)-alkoxycarbonyl wie Z, Fmoc oder Bpoc,

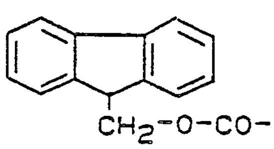

Fmoc

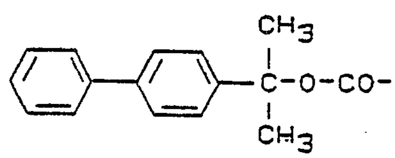

Bpoc

substituierte Z-Reste wie Moc, Ddz und Z (p-$NO_2$)

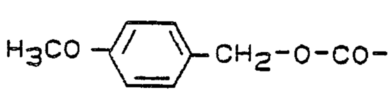

Moc

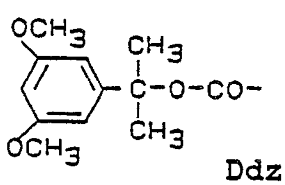

Ddz

und modifizierte Z-Reste wie Pyoc und entsprechende von 2- bzw. 3-Picolin abgeleitete Reste, die wie oben bei ($C_6$-$C_{12}$)-Aryl angegeben substituiert sein können.

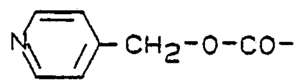

Pyoc

Bevorzugte $N^{im}$-Schutzgruppen sind solche, die in Gegenwart von Säuren, vorzugsweise in einem pH-Bereich von etwa 1 - 6 und/oder unter physiologischen Bedingungen abgespalten werden können.

Es ist überraschend, daß Verbindungen der Formel I mit $R^3$ = H vielfach stabiler sind als die entsprechenden Verbindungen mit $R^3$ = H. Sie sind vor allem stabiler unter sauren Bedingungen, wie sie beispielsweise im Magen herrschen, sowie in Gegenwart von Wasser. Durch gezielte Auswahl einer $N^{im}$-Schutzgruppe ist es somit für den Fachmann möglich, die Freisetzung der aktiven Verbindungen so zu steuern, daß diese selektiv am Wirkort erfolgt.

Gegebenenfalls vorhandene chirale C- und S-Atome können sowohl in der R- als auch in der S-Konfiguration vorkommen.

In solchen Fällen liegen Verbindungen der Formel I in Form der reinen Enantiomeren oder als Stereoisomerengemisch (wie Enantiomerengemisch und Diastereomerengemisch) vor.

Von besonderer Bedeutung sind die folgenden Verbindungen: 2-[4- Pyrrolidino-2-pyrimidinylmethylsulfinyl]-1H-thieno[3,4-d]imidazol,
2-[4-Piperidino-2-pyrimidinylmethylsulfinyl]-1H-thieno[3,4-d]imidazol,
2-[4-Morpholino-2-pyrimidinylmethylsulfinyl]-1H-thieno[3,4-d]imidazol,
2-[4-(N,N-Dimethylamino)-2-pyrimidinylmethylsulfinyl]-1H-thieno[3,4-d]imidazol,
2-[5-Methyl-4-pyrrolidino-2-pyrimidinylmethylsulfinyl]-1H-thieno[3,4-d]imidazol,
2-[5-Methyl-4-piperidino-2-pyrimidinylmethylsulfinyl]-1H-thieno[3,4-d]-imidazol,
2-[5-Methyl-4-morpholino-2-pyrimidinylmethylsulfinyl]-1H-thieno[3,4-d]imidazol,
2-[5-Methyl-4-(N,N-dimethylamino)-2-pyrimidinylmethylsulfinyl]-1H-thieno[3,4-d]imidazol,
2-[5-Amino-4-pyrrolidino-2-pyrimidinylmethylsulfinyl]-1H-thieno[3,4-d]imidazol,
2-[5-Amino-4-piperidino-2-pyrimidinylmethylsulfinyl]-1H-thieno[3,4-d]imidazol,
2-[5-Amino-4 morpholino-2-pyrimidinylmethylsulfinyl]-1H-thieno[3,4-d]imidazol,
2-[5-Amino-4-(N,N-dimethylamino)-2-pyrimidinylmethylsulfinyl]-1H-thieno[3,4-d]imidazol,
2-[5-Chlor-4-pyrrolidino-2-pyrimidinylmethylsulfinyl]-1H-thieno[3,4-d]imidazol,
2-[5-Chlor-4-piperidino-2-pyrimidinylmethylsulfinyl]-1H-thieno[3,4-d]imidazol,
2-[5-Chlor-4-morpholino-2 pyrimidinylmethylsulfinyl]-1H-thieno[3,4-d]imidazol,
2-[5-Chlor-4-(N,N-dimethylamino)-2-pyrimidinylmethylsulfinyl]-1H-thieno[3,4-d]imidazol,
2-[5-Chlor-6-pyrrolidino-4-pyrimidinylmethylsulfinyl]-1H-thieno[3,4-d]imidazol,
2-[5-Chlor-6-piperidino-4-pyrimidinylmethylsulfinyl]-1H-thieno[3,4-d]imidazol,
2-[5-Chlor-6-morpholino-4-pyrimidinylmethylsulfinyl]-1H-thieno[3,4-d]imidazol,
2-[5-Chlor-6-(N,N-dimethylamino)-4-pyrimidinylmethylsulfinyl]-1H-thieno[3,4-d]imidazol,
2-[6-Pyrrolidino-4-pyrimidinylmethylsulfinyl]-IH-thieno[3,4-d]imidazol,
2-[6-Piperidino-4-pyrimidinylmethylsulfinyl]-1H-thieno[3,4-d]imidazol,
2-[6-Morpholino-4-pyrimidinylmethylsulfinyl]-1H-thieno[3,4-d]imidazol,
2-[6-(N,N-Dimethylamino)-4-pyrimidinylmethylsulfinyl]-1H-thieno[3,4-d]imidazol,
2-[5-Methyl-6-pyrrolidino-4-pyrimidinylmethylsulfinyl]-1H-thieno[3,4-d]imidazol,
2-[5-Methyl-6-piperidino-4-pyrimidinylmethylsulfinyl]-1H-thieno[3,4-d]imidazol,
2-[5-Methyl-6-morpholino-4-pyrimidinylmethylsulfinyl]-1H-thieno[3,4-d]imidazol,
2-[5-Methyl-6-(N,N-dimethylamino)-4-pyrimidinylmethylsulfinyl]-1H-thieno[3,4-d]imidazol,
2-[5-Amino-6-pyrrolidino-4-pyrimidinylmethylsulfinyl]-1H-thieno[3,4-d]imidazol,
2-[5-Amino-6-piperidino-4-pyrimidinylmethylsulfinyl]-1H-thieno[3,4-d]imidazol,
2-[5-Amino-6-morpholino-4-pyrimidinylmethylsulfinyl]-1H-thieno[3,4-d]imidazol,
2-[5-Amino-6-(N,N-dimethylamino)-4-pyrimidinylmethylsulfinyl]-1H-thieno[3,4-d]imidazol, 2-[4-Methoxy-2-pyrimidinylmethylsulfinyl]-1H-thieno[3,4-d]imidazol,
2-[4-Ethoxy-2-pyrimidinylmethylsulfinyl]-1H-thieno[3,4-d]imidazol,
2-[4-Benzyloxy-2-pyrimidinylmethylsulfinyl]-1H-thieno[3,4-d]imidazol,
2-[4-Phenoxy-2-pyrimidinylmethylsulfinyl]-1H-thieno[3,4-d]imidazol,
2-[4-Isopropoxy-2-pyrimidinylmethylsulfinyl]-1H-thieno[3,4 d]imidazol,
2-[4-Methoxy-5-methyl-2-pyrimidinylmethylsulfinyl]-1H-thieno[3,4-d]imidazol,
2-[4-Ethoxy-5-methyl-2 pyrimidinylmethylsulfinyl]-1H-thieno[3,4-d]imidazol,
2-[4-Benzyloxy-5-methyl-2-pyrimidinylmethylsulfinyl]-1H-thieno[3,4-d]imidazol,

2-[5-Methyl-4-phenoxy-2-pyrimidinylmethylsulfinyl]-1H-thieno[3,4-d]imidazol,
2-[4-Isopropoxy-5-methyl-2-pyrimidinylmethylsulfinyl]-1H-thieno[3,4-d]imidazol,
2-[5-Chlor-4-methoxy-2-pyrimidinylmethylsulfinyl]-1H-thieno[3,4-d]imidazol,
2-[5-Chlor-4-ethoxy-2-pyrimidinylmethylsulfinyl]-1H-thieno[3,4-d]imidazol,
2-[5-Chlor-4-isopropoxy-2-pyrimidinylmethylsulfinyl]-1H-thieno[3,4-d]imidazol,
2-[4-Benzyloxy-5-chlor-2-pyrimidinylmethylsulfinyl]-1H-thieno[3,4-d]imidazol,
2-[5-Chlor-4-phenoxy-2-pyrimidinylmethylsulfinyl]-1H-thieno[3,4-d]imidazol,
2-[6-Methoxy-4-pyrimidinylmethylsulfinyl]-1H-thieno[3,4-d]imidazol,
2-[6-Ethoxy-4-pyrimidinylmethylsulfinyl]-1H-thieno[3,4-d]imidazol,
2-[6-Benzyloxy-4-pyrimidinylmethylsulfinyl]-1H-thieno[3,4-d]imidazol,
2-[6-Methoxy-5-methyl-4-pyrimidinylmethylsulfinyl]-1H-thieno[3,4-d]imidazol,
2-[6-Ethoxy-5-methyl-4-pyrimidinylmethylsulfinyl]-1H-thieno[3,4-d]imidazol,
2-[6-Benzyloxy-5-methyl-4-pyrimidinylmethylsulfinyl]-1H-thieno[3,4-d]imidazol, 2-[5-Chlor-6-methoxy-4-pyrimidinylmethylsulfinyl]-1H-thieno[3,4-d]imidazol,
2-[5-Chlor-6-ethoxy-4-pyrimidinylmethylsulfinyl]-1H-thieno[3,4-d]imidazol,
2-[4-Methylthio-2-pyrimidinylmethylsulfinyl]-1H-thieno[3,4-d]imidazol,
2-[4-Ethylthio-2-pyrimidinylmethylsulfinyl]-1H-thieno[3,4-d]imidazol,
2 [5-Methyl-4-methylthio-2-pyrimidinylmethylsulfinyl]-1H-thieno[3,4-d]imidazol,
2-[5-Methyl 4-ethylthio-2-pyrimidinylmethylsulfinyl]-1H-thieno[3,4-d]imidazol,
2-[5-Chlor-4-methylthio-2-pyrimidinylmethylsulfinyl]-1H-thieno[3,4-d]imidazol,
2-[5-Chlor-4-ethylthio-2-pyrimidinylmethylsulfinyl]-1H-thieno[3,4-d]imidazol,
2-[6-Methylthio-4-pyrimidinylmethylsulfinyl]-1H-thieno[3,4-d]imidazol,
2-[6-Ethylthio-4-pyrimidinylmethylsulfinyl]-1H-thieno[3,4-d]imidazol,
2-[5-Methyl-6-methylthio-4-pyrimidinylmethylsulfinyl]-1H-thieno[3,4-d]imidazol,
2-[6-Ethylthio-5-methyl-4-pyrimidinylmethylsulfinyl]-1H-thieno[3,4-d]imidazol,
2-[5-Chlor-6-methylthio-4-pyrimidinylmethylsulfinyl]-1H-thieno[3,4-d]imidazol,
2-[5-Chlor-6-ethylthio-4-pyrimidinylmethylsulfinyl]-1H-thieno[3,4-d]imidazol,
2-(5-Trifluormethyl-4-pyrrolidino-2-pyrimidinylmethylsulfinyl)-1H-thieno[3,4-d]imidazol,
2-(5-Trifluormethyl-4-piperidino-2-pyrimidinylmethylsulfinyl)-1H-thieno[3,4-d]imidazol,
2-(5-Trifluormethyl-4-morpholino-2-pyrimidinylmethylsulfinyl)-1H-thieno[3,4-d]imidazol,
2-[5-Trifluormethyl-4-(N,N-dimethylamino)-2-pyrimidinylmethylsulfinyl]-1H-thieno[3,4-d]imidazol,
2-(5-Fluor-4-pyrrolidino-2-pyrimidinylmethylsulfinyl)-1H-thieno[3,4-d]imidazol,
2-(5-Fluor-4-piperidino-2-pyrimidinylmethylsulfinyl)-1H-thieno[3,4-d]imidazol,
2-(5-Fluor-4-morpholino-2-pyrimidinylmethylsulfinyl)-1H-thieno[3,4-d]imidazol,
2-[5-Fluor-4-(N,N-dimethylamino)-2-pyrimidinylmethylsulfinyl]-1H-thieno[3,4-d]imidazol,
2-(5-Cyano-4-pyrrolidino 2-pyrimidinylmethylsulfinyl)-1H-thieno[3,4-d]imidazol,
2-(5-Cyano-4-piperidino-2-pyrimidinylmethylsulfinyl)-1H-thieno[3,4-d]imidazol,
2-(5-Fluor-6-pyrrolidino-4-pyrimidinylmethylsulfinyl)-1H-thieno[3,4-d]imidazol,
2-(5-Fluor-6-piperidino-4-pyrimidinylmethylsulfinyl)-1H-thieno[3,4-d]imidazol,
2-(5-Fluor-6-morpholino-4-pyrimidinylmethylsulfinyl)-1H-thieno[3,4-d]imidazol,
2-[5-Fluor-6-(N,N-dimethylamino)-4-pyrimidinylmethylsulfinyl]-1H-thieno[3,4-d]imidazol,
2-(5-Cyano-6-pyrrolidino-4-pyrimidinylmethylsulfinyl)-1H-thieno[3,4-d]imidazol,
2-(5-Cyano-6-piperidino-4-pyrimidinylmethylsulfinyl)-1H-thieno[3,4-d]imidazol,
2-(5-Cyano-6-morpholino-4-pyrimidinylmethylsulfinyl)-1H-thieno[3,4-d]imidazol,
2-[5-Cyano-6-(N,N-dimethylamino)-4-pyrimidinylmethylsulfinyl]-1H-thieno[3,4-d]imidazol,
2-(5-Nitro-6-pyrrolidino-4-pyrimidinylmethylsulfinyl)-1H-thieno[3,4-d]imidazol,
2-(5-Nitro-6-piperidino-4-pyrimidinylmethylsulfinyl)-1H-thieno[3,4-d]imidazol,
2-(5-Nitro-6-morpholino-4-pyrimidinylmethylsulfinyl)-1H-thieno[3,4-d]imidazol,
2-[5-Nitro-6-(N,N-dimethylamino)-4-pyrimidinylmethylsulfinyl]-1H-thieno[3,4-d]imidazol,
2-(5-Cyano-4-morpholino-2-pyrimidinylmethylsulfinyl)-1H-thieno[3,4-d]imidazol,
2-[5-Cyano-4-(N,N-dimethylamino)-2-pyrimidinylmethylsulfinyl]-1H-thieno[3,4-d]imidazol,
2-(5-Nitro-4-pyrrolidino-2-pyrimidinylmethylsulfinyl)-1H-thieno[3,4-d]imidazol,
2-(5-Nitro-4-piperidino-2-pyrimidinylmethylsulfinyl)-1H-thieno[3,4-d]imidazol,
2-(5-Nitro-4-morpholino-2-pyrimidinylmethylsulfinyl)-1H-thieno[3,4-d]imidazol,
2-[5-Nitro-4-(N,N-dimethylamino)-2-pyrimidinylmethylsulfinyl]-1H-thieno[3,4-d]imidazol,
2-(5-Trifluormethyl-6-pyrrolidino-4-pyrimidinylmethylsulfinyl)-1H-thieno[3,4-d]imidazol,
2-(5-Trifluormethyl-6-piperidino-4-pyrimidinylmethylsulfinyl)-1H-thieno[3,4-d]imidazol,
2-(5-Trifluormethyl-6-morpholino-4-pyrimidinylmethylsulfinyl)-1H-thieno[3,4-d]imidazol,

2-[5-Trifluormethyl-6-(N,N-dimethylamino)-4-pyrimidinylmethylsulfinyl]-1H-thieno[3,4-d]imidazol.

Die Erfindung betrifft weiterhin ein Verfahren zur Herstellung von Verbindungen der Formel I, das dadurch gekennzeichnet ist, daß man

a) eine Verbindung der Formel II

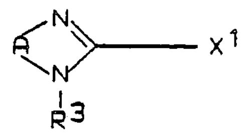

(II)

in welcher A, $R^1$, $R^2$ und $R^3$ wie auf den Seiten 1 und 2 definiert sind und

$X^1$ i. eine Abgangsgruppe oder

ii. -SH, $-S^-M^+$ oder $-SO_2^-M^+$ bedeutet, wobei $M^+$ für ein Kation, beispielsweise ein Alkali-, Erdalkali-, Ammonium oder Alkylammonium-Ion, insbesondere ein Natrium- oder Kalium-Ion, steht,

umsetzt mit einer Verbindung der Formel III

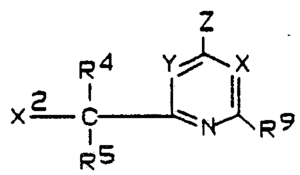

(III)

in welcher X, Y, Z, $R^4$, $R^5$, $R^6$, $R^7$, $R^8$, $R^9$ und $R^{10}$ wie auf den Seiten 2 und 3 definiert sind und

$X^2$ im oben genannten Fall i. -SH, $-S^-M^+$ oder $-SO_2^-M^+$ wobei $M^+$ für ein Kation steht, und im oben genannten Fall ii. eine Abgangsgruppe bedeutet, oder

b) eine Verbindung der Formel IV,

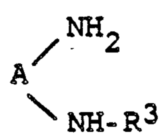

(IV)

in welcher A, $R^1$, $R^2$ und $R^3$ wie oben definiert sind, umsetzt mit einer Verbindung der Formel V

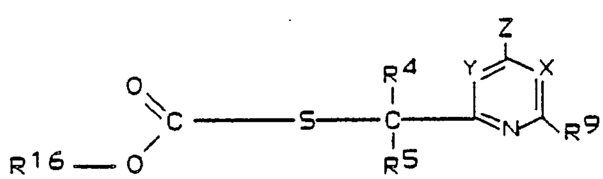

(V)

in welcher X, Y, Z, $R^4$, $R^5$, $R^6$, $R^7$, $R^8$, $R^9$ und $R^{10}$ wie oben definiert sind und $R^{16}$ für eine veresternde Gruppe steht,

i. in einer Verbindung der Formel I (eine) gegebenenfalls vorhandene -S-Gruppe(n) gewünschtenfalls zu(r) -SO oder $-SO_2$-Gruppe(n) oxidiert,

ii. in einer Verbindung der Formel I (eine) gegebenenfalls vorhandene -SO-Gruppe(n) gewünschtenfalls zu(r) $-SO_2$-Gruppe(n) oxidiert,

iii. eine Verbindung der Formel I, worin $R^3$ für Wasserstoff steht, gewünschtenfalls acyliert, alkyliert oder aralkyliert,

iv. eine Verbindung der Formel I, worin $R^3$ nicht Wasserstoff bedeutet, gewünschtenfalls verseift und

v. eine Verbindung der Formel I gewünschtenfalls in ihr physiologisch verträgliches Salz überführt,

wobei zwei oder mehr der Maßnahmen i.-iv. auch in einer anderen als der angegebenen Reihenfolge ausgeführt werden können.

Setzt man gemäß der hier bevorzugten Verfahrensvariante (a) Verbindungen der Formel II mit Verbindungen der Formel III um, so steht $X^1$ oder $X^2$ für eine Abgangsgruppe, die nucleophil ablösbar ist, wie Cl, Br, I, $-O-SO_2-CH_3$, $-O-SO_2-CF_3$ oder $-O-SO_2-(C_6H_4-pCH_3)$.

Die Umsetzung einer Verbindung der Formel II mit einer Verbindung der Formel III oder deren Salzen erfolgt in einem inerten Lösungsmittel wie z.B. Wasser, Methylenchlorid, Methanol, Ethanol, Aceton, Essigsäureethylester, Toluol, Tetrahydrofuran, Acetonitril, Dimethylformamid, Dimethylsulfoxid oder Gemischen dieser Lösungsmittel zweckmäßigerweise in Gegenwart einer anorganischen oder organischen Base, wie z.B. Natrium- oder Kaliumhydroxid, -carbonat, -alkoxid, -hydrid, -amid, Ammoniak, Triethylamin, Tributylamin, Pyridin bei -20 bis +150 °C, vorzugsweise bei 0 - 80 °C.

Die Verbindungen der Formel II können in Analogie zu bekannten Verfahren hergestellt werden, z.B. durch Ringschluß entsprechend substituierter 2,3-, 3,4- oder 4,5-Diaminothiophene der oben definierten Formel IV mit entsprechenden Schwefelverbindungen wie Schwefelkohlenstoff (z.B DE-A-31 32 167).

Die hierfür benötigten 2,3-, 3,4- oder 4,5-Diaminothiophene sind entweder literaturbekannt oder können in Analogie zu bekannten Verfahren hergestellt werden. Sie werden z.B. durch Reduktion entsprechend substituierter Aminonitrothiophene erhalten.

In den bei Verfahrensvariante (b) eingesetzten Estern der Formel V steht $R^{16}$ für eine veresternde Gruppe, vorzugsweise ($C_1$-$C_6$)-Alkyl oder Benzyl.

Die Umsetzung einer Verbindung der Formel IV mit einer Verbindung der Formel V gemäß Verfahrensvariante (b) erfolgt analog der in Preston et al., Benzimidazoles and Congeneric Tricyclic Compounds, Part 1, New York, Seiten 10-13 beschriebenen Verfahrensweisen.

Die so erhaltenen Verbindungen der Formel I können, falls $R^3$ Wasserstoff bedeutet, in physiologisch verträgliche Salze umgewandelt werden.

Verbindungen der Formel I mit T = -S- können ferner mit geeigneten Oxidationsmitteln in solche mit T = -SO- oder -$SO_2$- umgewandelt werden. In gleicher Weise lassen sich auch -S-Gruppen in den Substituenten $R^1$, $R^2$ und $R^6$ bis $R^9$ oxidieren.

Diese Reaktion erfolgt in einem geeigneten, inerten Lösungsmittel wie z.B. Methylenchlorid, Chloroform, Tetrachlorkohlenstoff, 1,2-Dichlorethan, Toluol, Essigsäureethylester, Essigsäure, Trifluoressigsäure, Wasser, Methanol, Ethanol oder Gemischen derselben bei -20 °C bis +150 °C vorzugsweise bei -10 °C bis +40 °C.

Als Oxidationsmittel kommen z.B. in Betracht: Wasserstoffperoxid, Persäuren und Perester wie Peressigsäure, Trifluorperessigsäure, Monoperphthalsäure, m-Chlorperbenzoesäure und deren Ester, Ozon, Distickstofftetroxid, Iodosobenzol, N-Chlorsuccinimid, 1-Chlorbenzotriazol, Natriumhypochlorit, Kaliumperoxodisulfat, t-Butylhypochlorit, Tetrabutylammoniumperjodat oder -permanganat, Natrium-meta-perjodat, Selen- oder Mangandioxid, Cerammonnitrat, Chromsäure, Chlor, Brom, Diazabicyclo-[2.2.2]octan-Bromkomplex, Dioxandibromid, Pyridiniumbromidperbromid, Sulfurylchlorid, 2-Arylsulfonyl-3-aryloxaziridine, Titantetraisopropylat/tert. Butylhydroperoxid (gegebenenfalls unter Zusatz von Dialkylestern der (D)- bzw. (L)-Weinsäure und einer definierten Menge Wasser).

Ebenso können isolierte, ggf. immobilisierte oxidierende Enzyme oder Mikroorganismen als Oxidationsmittel Anwendung finden.

Die Oxidationsmittel werden in äquimolaren Mengen, ggf. auch in einem geringen Überschuß von 5 -10 Mol-% bei der Oxidation zu T = -SO- oder auch in größerem Überschuß und/oder bei höherer Reaktionstemperatur eingesetzt wenn eine Oxidation zu T = -$SO_2$- gewünscht wird.

Verbindungen der Formel I mit $R^3 \neq H$ können hergestellt werden ausgehend von Verbindungen der Formel IV mit $R^3 \neq H$ und Verbindungen der Formel V oder durch Acylierung, Alkylierung oder Aralkylierung von Verbindungen der Formel I mit $R^3$ = H. Im folgenden soll auf den zweiten Weg etwas näher eingegangen werden.

Die Acylierung, Alkylierung oder Aralkylierung von Verbindungen der Formel I wird in an sich bekannter Weise mit den entsprechenden Acylierungsmitteln, Alkylierungsmitteln bzw. Aralkylierungsmitteln in einem geeigneten organischen Lösungsmittel in der Regel bei einer Temperatur zwischen -78 °C und dem Siedepunkt des Reaktionsgemisches gegebenenfalls in Gegenwart einer Base durchgeführt.

$N^{im}$-Schutzgruppen der Formel VI mit p = 0, q = 1, W = Bindung und B = Wasserstoff lassen sich in Verbindungen der Formel I ($R^3$ = H, T = S) beispielsweise durch Hydroxyalkylierung einführen, wobei man $N^{im}$-Schutzgruppen mit $R^{11}$ = $R^{12}$ = Wasserstoff in an sich bekannter Weise (vgl. z.B. Eur. J. Med. Chem. 15 [1980] 586; J. Med. Chem. 22 [1979] 1113) durch Hydroxymethylierung mit Formaldehyd in einem organischen Lösungsmittel wie z.B. Acetonitril einführen kann. Die Hydroxyalkylierung wird bei einer Temperatur zwischen 0 °C und dem Siedepunkt des Reaktionsgemisches gegebenenfalls in Gegenwart einer Base wie Triethylamin durchgeführt.

Hydroxymethylverbindungen der Formel VII

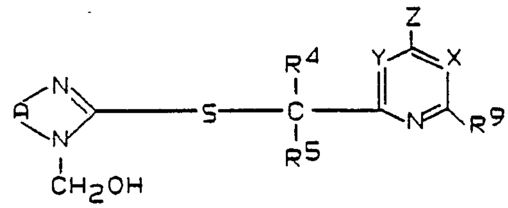

(VII)

können in der in EP-A-176308, Seite 11 beschriebenen Weise in Acylderivate der Formel VIII

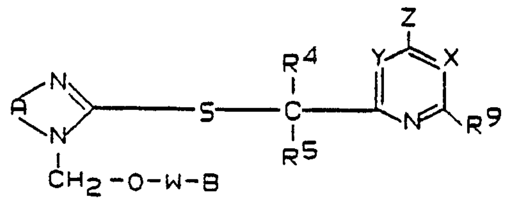

(VIII)

worin W-B ein Acylrest ist, überführt werden.

Verbindungen der Formel I mit $R^3$ = H können auch mit Reagenzien der Formel IX

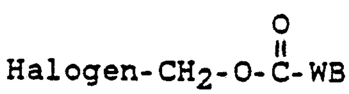

(IX)

wie z.B. Chlormethylpivalat, in bekannter Weise alkyliert werden, wobei die entsprechenden Carbonate (W = -CO-O- oder -CO-O-$CR^{13}R^{14}$-) erhalten werden. Die Umsetzung wird z.B. in der im EP-A-176308, Seite 12 beschriebenen Weise durchgeführt.

Acylreste von Aminosäuren werden in bekannter Weise (z.B. DCC/HOBt- oder Dialkylphosphinsäureanhydrid-Methode) an Verbindungen der Formel I mit $R^3$ = H gekuppelt.

$N^{im}$-Schutzgruppen der Formel VI mit p = 0, q = 1 und $R^{11}$ und/oder $R^{12}$ ≠ Wasserstoff werden eingeführt, indem man eine Verbindung der Formel I ($R^3$ = H, T = S) mit 1 bis 10 Äquivalenten, vorzugsweise 2 bis 3 Äquivalenten des entsprechenden α-Halogenalkylesters umsetzt. Die verwendeten α-Halogenalkylester werden aus Säurehalogeniden und Aldehyden nach bekannten Methoden erhalten (vgl. z.B. J. Amer. Chem. Soc. 43 [1921] 660; J. Med. Chem. 23 [1980] 469-474).

Bevorzugt werden Bromalkylester verwendet. Alternativ kann man das Anion einer Verbindung der Formel I ($R^3$ = H, T = S), welches aus dieser und NaH zugänglich ist, mit dem α-Halogenalkylester behandeln.

An Stelle der α-Halogenalkylester können auch [1-(Alkylcarbonyloxy)-alkyl]-pyridinium-Salze, die analog den bekannten [1-(Arylcarbonyloxy)-alkyl]-pyridinium-Salzen (vgl. Angew. Chem. Suppl. 1982, 675-685) aus den entsprechenden Acylhalogeniden, Aldehyden und Pyridin hergestellt werden, Verwendung finden.

Alkylaminoacetale der Formel I, worin $R^3$ für einen Rest der Formel VI steht, in dem p = 0, q = 1 ist und W eine Bindung oder -$CR^{13}R^{14}$- bedeutet und B obige Bedeutung hat, werden hergestellt, indem man eine Verbindung der obengenannten Formel VI in einem dipolar aprotischen Lösungsmittel wie Dimethylformamid bei etwa 20 bis 50° C, vorzugsweise bei etwa 25° C mit etwa einem Äquivalent NaH behandelt. Das so erhaltene Anion wird dann mit etwa einem Äquivalent eines Halogenethers der Formel Halogen-$CR^{11}R^{12}$-W-B (Halogen = Chlor oder Brom) umgesetzt, wobei man die Reaktionsmischung 15 Minuten lang bei etwa 20 bis 50° C, vorzugsweise bei ca. 25° C rührt. Die Halogenether sind bekannt und sind vielfach im Handel erhältlich oder sie können in Analogie zu bekannten Verbindungen hergestellt werden.

Urethane der Formel I, worin $R^3$ für eine Urethanschutzgruppe der Formel VI (p = 1, q = 0 und W = Bindung oder -$CR^{13}R^{14}$-) steht, werden aus den entsprechenden Verbindungen mit $R^3$ = H erhalten, in dem man diese gegebenenfalls in Gegenwart einer Base, wie NaH, in einem geeigneten Lösungsmittel, wie DMF, mit Fluor- oder Chlorameisensäureestern der Formel Cl(F)-CO-O-WB umsetzt (in Analogie zu der in EP-A-176308, Seite 12 beschriebenen Verfahrensweise).

Die Fluor- bzw. Chlorformiate sind bekannt und oft im Handel erhältlich oder können nach bekannten Methoden hergestellt werden.

Aralkyloxycarbonyl- und Alkoxycarbonylgruppen können auch mit den bekannten, oft käuflichen Dicarbonaten, wie Di-tert. Butyldicarbonat, Dibenzyldicarbonat, eingeführt werden.

Substituierte bzw. modifizierte Z-Gruppen in denen $R^{13}$ und/oder $R^{14}$ = Wasserstoff sind, werden durch Reaktion der entsprechenden ungeschützten Verbindung der Formel I, wenn notwendig, unter Zuhilfenahme einer Base mit den entsprechenden Aziden oder den entsprechenden Carbonaten hergestellt.

Zur Acylierung der Verbindungen der Formel I ($R^3$ = H, T = S) können neben den üblichen Standardbedingungen (z.B. Acetanhydrid, Triethylamin, Dimethylaminopyridin) auch andere Verfahren, wie z.B. Umsetzung mit N-[1-(Arylcarbonyloxy)-alkyl]-pyridinium-Salzen (bekannt aus Angew. Chem. Suppl. 1982, 675-685) angewandt werden.

Zur Herstellung von Dialkoxyderivaten der Formel I ($R^3$ = $-CR^{13}R^{14}-B$, worin $R^{13}$ und $R^{14}$ jeweils Alkoxy oder zusammen Alkylendioxy und B = H bedeuten; T = S oder SO) wird vorzugsweise die entsprechende Verbindung der Formel I mit $R^3$ = H in Gegenwart einer Base mit den entsprechenden Orthoameisensäureestern, wie Orthoameisensäuretrialkylestern, umgesetzt.

Verbindungen der Formel III in denen $X^2$ Hydroxy ist, können durch Reaktion entsprechend substituierter Amidine mit geeigneten substituierten β-Keto-Estern hergestellt werden. Zum Beispiel ergibt die Umsetzung von Methoxyacetamidin-Hydrochlorid mit dem Natriumsalz von Ethylformylacetat die Verbindung der Formel III (Y = N, X = CH, $X^2$ = $OCH_3$, Z = OH).

Analog führt die Reaktion von Formamidinacetat mit 3-Keto-4-methoxybuttersäuremethylester zur Verbindung der Formel III (Y = CH, X = N, $X^2$ = $OCH_3$, Z = OH).

Erfindungsgemäß können außer den in den Ausführungsbeispielen beschriebenen Thienoimidazol-Derivaten beispielsweise auch die in der folgenden Tabelle zusammengestellten Verbindungen der allgemeinen Formel I bzw. deren Salze erhalten werden.

Verwendete Abkürzungen:

Methyl (Me), Ethyl (Et), Propyl (Pr), Butyl (Bu), Hexyl (Hex), Acetyl (Ac), Phenyl (Ph), cyclo (c), iso (i).

Tabelle

(T= SO; A wie oben unter (b) definiert; $R^4$, $R^5$, $R^9$ jeweils= Wasserstoff)

| $R^1$ | $R^2$ | $R^3$ | $R^6$ | X | Y | Z | $R^{10}$ |
|---|---|---|---|---|---|---|---|
| Me | Me | H | Cl | N | $CR^6$ | $OR^{10}$ | Me |
| Me | Me | H | Cl | N | $CR^6$ | $OR^{10}$ | Et |
| Me | Me | H | Cl | N | $OR^6$ | $OR^{10}$ | i-Pr |
| Me | Me | H | Cl | N | $OR^6$ | $OR^{10}$ | $CH_2Ph$ |
| Me | Me | H | Cl | N | $OR^6$ | $OR^{10}$ | Ph |
| Me | Me | H | Cl | $OR^6$ | N | $OR^{10}$ | Me |
| Me | Me | H | Cl | $OR^6$ | N | $OR^{10}$ | Et |
| Me | Me | H | Cl | $OR^6$ | N | $OR^{10}$ | i-Pr |
| Me | Me | H | Cl | $OR^6$ | N | $OR^{10}$ | $CH_2Ph$ |
| Me | Me | H | Cl | $OR^6$ | N | $OR^{10}$ | Ph |
| Me | Me | H | $CH_3$ | N | $CR^6$ | $OR^{10}$ | Me |
| Me | Me | H | $CH_3$ | N | $CR^6$ | $OR^{10}$ | Et |
| Me | Me | H | $CH_3$ | N | $CR^6$ | $OR^{10}$ | i-Pr |
| Me | Me | H | $CH_3$ | N | $CR^6$ | $OR^{10}$ | Ph |
| Me | Me | H | $CH_3$ | N | $CR^6$ | $OR^{10}$ | $CH_2Ph$ |
| Me | Me | H | Me | $CR^6$ | N | $OR^{10}$ | Me |
| Me | Me | H | Me | $CR^6$ | N | $OR^{10}$ | Et |
| Me | Me | H | Me | $CR^6$ | N | $OR^{10}$ | i-Pr |
| Me | Me | H | Me | $CR^6$ | N | $OR^{10}$ | $CH_2Ph$ |
| Me | Me | H | Me | $CR^6$ | N | $OR^{10}$ | Ph |
| Me | Me | H | H | $CR^6$ | N | $OR^{10}$ | Me |
| Me | Me | H | H | $CR^6$ | N | $OR^{10}$ | Et |
| Me | Me | H | H | $CR^6$ | N | $OR^{10}$ | i-Pr |
| Me | Me | H | H | $CR^6$ | N | $OR^{10}$ | $CH_2Ph$ |
| Me | Me | H | H | $CR^6$ | N | $OR^{10}$ | Ph |
| Me | Me | H | H | N | $CR^6$ | $OR^{10}$ | Me |
| Me | Me | H | H | N | $CR^6$ | $OR^{10}$ | Et |
| Me | Me | H | H | N | $CR^6$ | $OR^{10}$ | i-Pr |

| $R^1$ | $R^2$ | $R^3$ | $R^6$ | X | Y | Z | $R^{10}$ |
|---|---|---|---|---|---|---|---|
| Me | Me | H | H | N | $CR^6$ | $OR^{10}$ | $Ch_2Ph$ |
| Me | Me | H | H | N | $CR^6$ | $OR^{10}$ | Ph |
| H | H | H | Cl | $CR^6$ | N | $OR^{10}$ | Me |
| H | H | H | Cl | $CR^6$ | N | $OR^{10}$ | Et |
| H | H | H | Cl | $CR^6$ | N | $OR^{10}$ | i-Pr |
| H | H | H | Cl | $CR^6$ | N | $OR^{10}$ | $CH_2Ph$ |
| H | H | H | Cl | $CR^6$ | N | $OR^{10}$ | Ph |
| H | H | H | Cl | N | $CR^6$ | $OR^{10}$ | Me |
| H | H | H | Cl | N | $CR^6$ | $OR^{10}$ | Et |
| H | H | H | Cl | N | $CR^6$ | $OR^{10}$ | i-Pr |
| H | H | H | Cl | N | $CR^6$ | $OR^{10}$ | $CH_2Ph$ |
| H | H | H | Cl | N | $CR^6$ | $OR^{10}$ | Ph |
| H | H | H | H | $CR^6$ | N | $OR^{10}$ | Me |
| H | H | H | H | $CR^6$ | N | $OR^{10}$ | Et |
| H | H | H | H | $CR^6$ | N | $OR^{10}$ | i-Pr |
| H | H | H | H | $CR^6$ | N | $OR^{10}$ | $CH_2Ph$ |
| H | H | H | H | $CR^6$ | N | $OR^{10}$ | Ph |
| H | H | H | H | N | $CR^6$ | $OR^{10}$ | Me |
| H | H | H | H | N | $CR^6$ | $OR^{10}$ | Et |
| H | H | H | H | N | $CR^6$ | $OR^{10}$ | i-Pr |
| H | H | H | H | N | $CR^6$ | $OR^{10}$ | $CH_2Ph$ |
| H | H | H | H | N | $CR^6$ | $OR^{10}$ | Ph |
| Me | Me | H | Me | N | $CR^6$ | $SR^{10}$ | H |
| Me | Me | H | Me | N | $CR^6$ | $SR^{10}$ | Me |
| Me | Me | H | Me | N | $CR^6$ | $SR^{10}$ | Et |
| Me | Me | H | Me | N | $CR^6$ | $SR^{10}$ | i-Pr |
| Me | Me | H | Me | N | $CR^6$ | $SR^{10}$ | Ph |
| Me | Me | H | Me | $CR^6$ | N | $SR^{10}$ | H |
| Me | Me | H | Me | $CR^6$ | N | $SR^{10}$ | Me |
| Me | Me | H | Me | $CR^6$ | N | $SR^{10}$ | Et |
| Me | Me | H | Me | $CR^6$ | N | $SR^{10}$ | i-Pr |
| Me | Me | H | Me | $CR^6$ | N | $SR^{10}$ | Ph |

| $R^1$ | $R^2$ | $R^3$ | $R^6$ | X | Y | Z | $R^{10}$ |
|---|---|---|---|---|---|---|---|
| H | H | H | Me | N | $CR^6$ | $SR^{10}$ | H |
| H | H | H | Me | N | $CR^6$ | $SR^{10}$ | Me |
| H | H | H | Me | N | $CR^6$ | $SR^{10}$ | Et |
| H | H | H | Me | N | $CR^6$ | $SR^{10}$ | i-Pr |
| H | H | H | Me | N | $CR^6$ | $SR^{10}$ | Ph |
| H | H | H | Me | $CR^6$ | N | $SR^{10}$ | H |
| H | H | H | Me | $CR^6$ | N | $SR^{10}$ | Me |
| H | H | H | Me | $CR^6$ | N | $SR^{10}$ | Et |
| H | H | H | Me | $CR^6$ | N | $SR^{10}$ | i-Pr |
| H | H | H | Me | $CR^6$ | N | $SR^{10}$ | Ph |
| H | H | H | H | N | $CR^6$ | $SR^{10}$ | H |
| H | H | H | H | N | $CR^6$ | $SR^{10}$ | Me |
| H | H | H | H | N | $CR^6$ | $SR^{10}$ | Et |
| H | H | H | H | N | $CR^6$ | $SR^{10}$ | i-Pr |
| H | H | H | H | N | $CR^6$ | $SR^{10}$ | Ph |
| H | H | H | H | $CR^6$ | N | $SR^{10}$ | H |
| H | H | H | H | $CR^6$ | N | $SR^{10}$ | Me |
| H | H | H | H | $CR^6$ | N | $SR^{10}$ | Et |
| H | H | H | H | $CR^6$ | N | $SR^{10}$ | i-Pr |
| H | H | H | H | $CR^6$ | N | $SR^{10}$ | Ph |
| Me | Me | H | H | N | $CR^6$ | $SR^{10}$ | H |
| Me | Me | H | H | N | $CR^6$ | $SR^{10}$ | Me |
| Me | Me | H | H | N | $CR^6$ | $SR^{10}$ | Et |
| Me | Me | H | H | N | $CR^6$ | $SR^{10}$ | i-Pr |
| Me | Me | H | H | N | $CR^6$ | $SR^{10}$ | Ph |
| Me | Me | H | H | $CR^6$ | N | $SR^{10}$ | H |
| Me | Me | H | H | $CR^6$ | N | $SR^{10}$ | Me |
| Me | Me | H | H | $CR^6$ | N | $SR^{10}$ | Et |
| Me | Me | H | H | $CR^6$ | N | $SR^{10}$ | i-Pr |
| Me | Me | H | H | $CR^6$ | N | $SR^{10}$ | Ph |

| $R^1$ | $R^2$ | $R^3$ | $R^6$ | X | Y | Z |
|---|---|---|---|---|---|---|
| H | H | H | H | $CR^6$ | N | $-NH_2$ |
| H | H | H | H | $CR^6$ | N | -NHMe |
| H | H | H | H | $CR^6$ | N | -NHEt |
| H | H | H | H | $CR^6$ | N | -NHPh |
| H | H | H | H | $CR^6$ | N | $-NH-CH_2Ph$ |
| H | H | H | H | $CR^6$ | N | -NH- 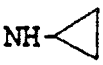 |
| H | H | H | H | $CR^6$ | N | -NH-i-Pr |
| H | H | H | H | $CR^6$ | N | -NH- 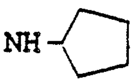 |
| H | H | H | H | $CR^6$ | N | -NH- 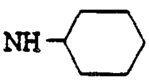 |
| H | H | H | H | $CR^6$ | N | $-NMe_2$ |
| H | H | H | H | $CR^6$ | N | $-NEt_2$ |
| H | H | H | H | $CR^6$ | N | $-N(iPr)_2$ |
| H | H | H | H | $CR^6$ | N | 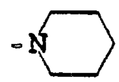 |
| H | H | H | H | $CR^6$ | N | 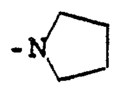 |
| H | H | H | H | $CR^6$ | N | 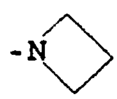 |
| H | H | H | H | $CR^6$ | N | 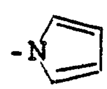 |
| H | H | H | H | $CR^6$ | N | 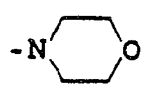 |
| H | H | H | H | $CR^6$ | N | 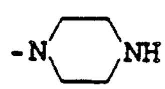 |

| $R^1$ | $R^2$ | $R^3$ | $R^6$ | X | Y | Z |
|---|---|---|---|---|---|---|
| H | H | H | H | N | $CR^6$ | $-NH_2$ |
| H | H | H | H | N | $CR^6$ | -NHMe |
| H | H | H | H | N | $CR^6$ | -NHEt |
| H | H | H | H | N | $CR^6$ | -NHPh |
| H | H | H | H | N | $CR^6$ | $-NH-CH_2Ph$ |
| H | H | H | H | N | $CR^6$ | -NH- 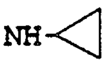 |
| H | H | H | H | N | $CR^6$ | -NH-i-Pr |
| H | H | H | H | N | $CR^6$ | -NH- 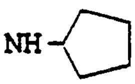 |
| H | H | H | H | N | $CR^6$ | -NH- 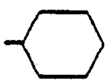 |
| H | H | H | H | N | $CR^6$ | $-NMe_2$ |
| H | H | H | H | N | $CR^6$ | $-NEt_2$ |
| H | H | H | H | N | $CR^6$ | $-N(iPr)_2$ |
| H | H | H | H | N | $CR^6$ | 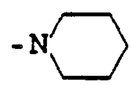 |
| H | H | H | H | N | $CR^6$ | 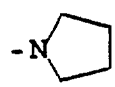 |
| H | H | H | H | N | $CR^6$ | 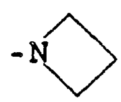 |
| H | H | H | H | N | $CR^6$ | 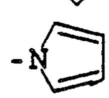 |
| H | H | H | H | N | $CR^6$ | 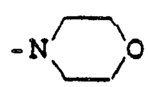 |
| H | H | H | H | N | $CR^6$ | 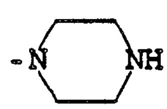 |

| $R^1$ | $R^2$ | $R^3$ | $R^6$ | X | Y | Z |
|---|---|---|---|---|---|---|
| H | H | H | Cl | $CR^6$ | N | $-NH_2$ |
| H | H | H | Cl | $CR^6$ | N | -NHMe |
| H | H | H | Cl | $CR^6$ | N | -NHEt |
| H | H | H | Cl | $CR^6$ | N | -NHPh |
| H | H | H | Cl | $CR^6$ | N | $-NH-CH_2Ph$ |
| H | H | H | Cl | $CR^6$ | N | 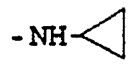 |
| H | H | H | Cl | $CR^6$ | N | -NH-i-Pr |
| H | H | H | Cl | $CR^6$ | N | 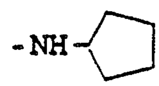 |
| H | H | H | Cl | $CR^6$ | N | 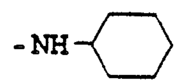 |
| H | H | H | Cl | $CR^6$ | N | $-NMe_2$ |
| H | H | H | Cl | $CR^6$ | N | $-NEt_2$ |
| H | H | H | Cl | $CR^6$ | N | $-N(iPr)_2$ |
| H | H | H | Cl | $CR^6$ | N | 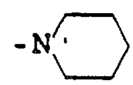 |
| H | H | H | Cl | $CR^6$ | N | 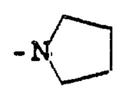 |
| H | H | H | Cl | $CR^6$ | N | 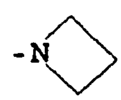 |
| H | H | H | Cl | $CR^6$ | N | 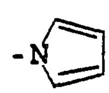 |
| H | H | H | Cl | $CR^6$ | N | 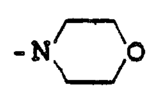 |
| H | H | H | Cl | $CR^6$ | N | 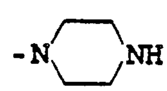 |

| $R^1$ | $R^2$ | $R^3$ | $R^6$ | X | Y | Z |
|---|---|---|---|---|---|---|
| H | H | H | Cl | N | $CR^6$ | $-NH_2$ |
| H | H | H | Cl | N | $CR^6$ | -NHMe |
| H | H | H | Cl | N | $CR^6$ | -NHEt |
| H | H | H | Cl | N | $CR^6$ | -NHPh |
| H | H | H | Cl | N | $CR^6$ | $-NH-CH_2Ph$ |
| H | H | H | Cl | N | $CR^6$ | -NH 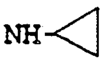 |
| H | H | H | Cl | N | $CR^6$ | -NH-i-Pr |
| H | H | H | Cl | N | $CR^6$ | -NH 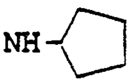 |
| H | H | H | Cl | N | $CR^6$ | -NH 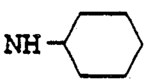 |
| H | H | H | Cl | N | $CR^6$ | $-NMe_2$ |
| H | H | H | Cl | N | $CR^6$ | $-NEt_2$ |
| H | H | H | Cl | N | $CR^6$ | $-N(iPr)_2$ |
| H | H | H | Cl | N | $CR^6$ | 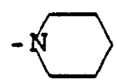 |
| H | H | H | Cl | N | $CR^6$ | 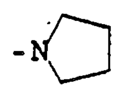 |
| H | H | H | Cl | N | $CR^6$ | 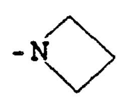 |
| H | H | H | Cl | N | $CR^6$ | 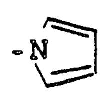 |
| H | H | H | Cl | N | $CR^6$ | 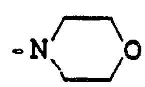 |
| H | H | H | Cl | N | $CR^6$ | 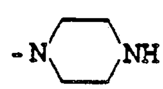 |

| $R^1$ | $R^2$ | $R^3$ | $R^6$ | X | Y | Z |
|---|---|---|---|---|---|---|
| H | H | H | i-Pr | $CR^6$ | N | $-NH_2$ |
| H | H | H | i-Pr | $CR^6$ | N | -NHMe |
| H | H | H | i-Pr | $CR^6$ | N | -NHEt |
| H | H | H | i-Pr | $CR^6$ | N | -NHPh |
| H | H | H | i-Pr | $CR^6$ | N | $-NH-CH_2Ph$ |
| H | H | H | i-Pr | $CR^6$ | N | -NH- 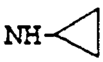 |
| H | H | H | i-Pr | $CR^6$ | N | -NH-i-Pr |
| H | H | H | i-Pr | $CR^6$ | N | -NH- 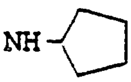 |
| H | H | H | i-Pr | $CR^6$ | N | -NH- 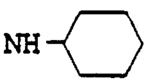 |
| H | H | H | i-Pr | $CR^6$ | N | $-NMe_2$ |
| H | H | H | i-Pr | $CR^6$ | N | $-NEt_2$ |
| H | H | H | i-Pr | $CR^6$ | N | $-N(iPr)_2$ |
| H | H | H | i-Pr | $CR^6$ | N | -N 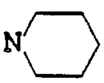 |
| H | H | H | i-Pr | $CR^6$ | N | -N 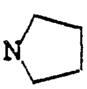 |
| H | H | H | i-Pr | $CR^6$ | N | -N 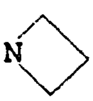 |
| H | H | H | i-Pr | $CR^6$ | N | -N 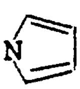 |
| H | H | H | i-Pr | $CR^6$ | N | -N 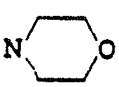 |
| H | H | H | i-Pr | $CR^6$ | N | -N 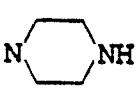 |

| $R^1$ | $R^2$ | $R^3$ | $R^6$ | X | Y | Z |
|---|---|---|---|---|---|---|
| H | H | H | i-Pr | N | $CR^6$ | $-NH_2$ |
| H | H | H | i-Pr | N | $CR^6$ | -NHMe |
| H | H | H | i-Pr | N | $CR^6$ | -NHEt |
| H | H | H | i-Pr | N | $CR^6$ | -NHPh |
| H | H | H | i-Pr | N | $CR^6$ | $-NH-CH_2Ph$ |
| H | H | H | i-Pr | N | $CR^6$ | -NH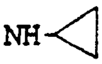 |
| H | H | H | i-Pr | N | $CR^6$ | -NH-i-Pr |
| H | H | H | i-Pr | N | $CR^6$ | -NH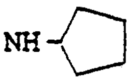 |
| H | H | H | i-Pr | N | $CR^6$ | -NH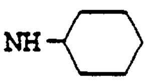 |
| H | H | H | i-Pr | N | $CR^6$ | $-NMe_2$ |
| H | H | H | i-Pr | N | $CR^6$ | $-NEt_2$ |
| H | H | H | i-Pr | N | $CR^6$ | $-N(iPr)_2$ |
| H | H | H | i-Pr | N | $CR^6$ | -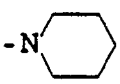 |
| H | H | H | i-Pr | N | $CR^6$ | -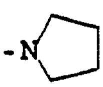 |
| H | H | H | i-Pr | N | $CR^6$ | -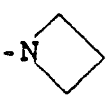 |
| H | H | H | i-Pr | N | $CR^6$ | -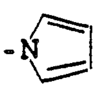 |
| H | H | H | i-Pr | N | $CR^6$ | -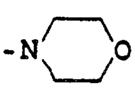 |
| H | H | H | i-Pr | N | $CR^6$ | -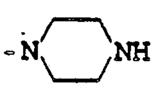 |

| $R^1$ | $R^2$ | $R^3$ | $R^6$ | X | Y | Z |
|---|---|---|---|---|---|---|
| H | H | H | Et | N | $CR^6$ | $-NH_2$ |
| H | H | H | Et | N | $CR^6$ | -NHMe |
| H | H | H | Et | N | $CR^6$ | -NHEt |
| H | H | H | Et | N | $CR^6$ | -NHPh |
| H | H | H | Et | N | $CR^6$ | $-NH-CH_2Ph$ |
| H | H | H | Et | N | $CR^6$ | -NH-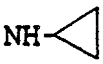 |
| H | H | H | Et | N | $CR^6$ | -NH-i-Pr |
| H | H | H | Et | N | $CR^6$ | -NH-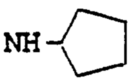 |
| H | H | H | Et | N | $CR^6$ | -NH-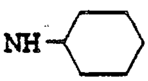 |
| H | H | H | Et | N | $CR^6$ | $-NMe_2$ |
| H | H | H | Et | N | $CR^6$ | $-NEt_2$ |
| H | H | H | Et | N | $CR^6$ | $-N(iPr)_2$ |
| H | H | H | Et | N | $CR^6$ | -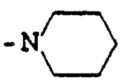 |
| H | H | H | Et | N | $CR^6$ | -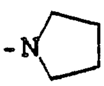 |
| H | H | H | Et | N | $CR^6$ | -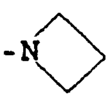 |
| H | H | H | Et | N | $CR^6$ | -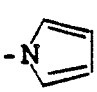 |
| H | H | H | Et | N | $CR^6$ | -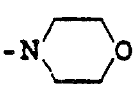 |
| H | H | H | Et | N | $CR^6$ | -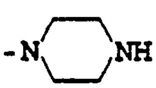 |

| $R^1$ | $R^2$ | $R^3$ | $R^6$ | X | Y | Z |
|---|---|---|---|---|---|---|
| H | H | H | Et | $CR^6$ | N | $-NH_2$ |
| H | H | H | Et | $CR^6$ | N | -NHMe |
| H | H | H | Et | $CR^6$ | N | -NHEt |
| H | H | H | Et | $CR^6$ | N | -NHPh |
| H | H | H | Et | $CR^6$ | N | $-NH-CH_2Ph$ |
| H | H | H | Et | $CR^6$ | N | -NH- 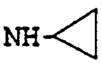 |
| H | H | H | Et | $CR^6$ | N | -NH-i-Pr |
| H | H | H | Et | $CR^6$ | N | -NH- 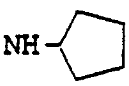 |
| H | H | H | Et | $CR^6$ | N | -NH- 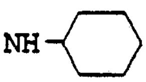 |
| H | H | H | Et | $CR^6$ | N | $-NMe_2$ |
| H | H | H | Et | $CR^6$ | N | $-NEt_2$ |
| H | H | H | Et | $CR^6$ | N | $-N(iPr)_2$ |
| H | H | H | Et | $CR^6$ | N | - 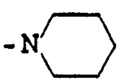 |
| H | H | H | Et | $CR^6$ | N | - 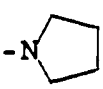 |
| H | H | H | Et | $CR^6$ | N | - 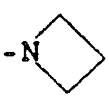 |
| H | H | H | Et | $CR^6$ | N | - 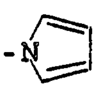 |
| H | H | H | Et | $CR^6$ | N | - 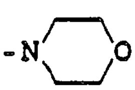 |
| H | H | H | Et | $CR^6$ | N | - 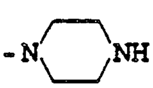 |

| $R^1$ | $R^2$ | $R^3$ | $R^6$ | X | Y | Z |
|---|---|---|---|---|---|---|
| H | H | H | Me | N | $CR^6$ | $-NH_2$ |
| H | H | H | Me | N | $CR^6$ | -NHMe |
| H | H | H | Me | N | $CR^6$ | -NHEt |
| H | H | H | Me | N | $CR^6$ | -NHPh |
| H | H | H | Me | N | $CR^6$ | $-NH-CH_2Ph$ |
| H | H | H | Me | N | $CR^6$ | -NH 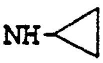 |
| H | H | H | Me | N | $CR^6$ | -NH-i-Pr |
| H | H | H | Me | N | $CR^6$ | -NH 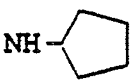 |
| H | H | H | Me | N | $CR^6$ | -NH 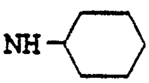 |
| H | H | H | Me | N | $CR^6$ | $-NMe_2$ |
| H | H | H | Me | N | $CR^6$ | $-NEt_2$ |
| H | H | H | Me | N | $CR^6$ | $-N(iPr)_2$ |
| H | H | H | Me | N | $CR^6$ | - 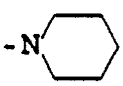 |
| H | H | H | Me | N | $CR^6$ | - 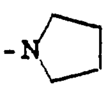 |
| H | H | H | Me | N | $CR^6$ | - 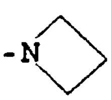 |
| H | H | H | Me | N | $CR^6$ | - 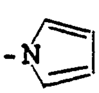 |
| H | H | H | Me | N | $CR^6$ | - 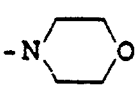 |
| H | H | H | Me | N | $CR^6$ | - 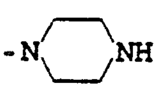 |

| $R^1$ | $R^2$ | $R^3$ | $R^6$ | X | Y | Z |
|---|---|---|---|---|---|---|
| H | H | H | Me | $CR^6$ | N | $-NH_2$ |
| H | H | H | Me | $CR^6$ | N | -NHMe |
| H | H | H | Me | $CR^6$ | N | -NHEt |
| H | H | H | Me | $CR^6$ | N | -NHPh |
| H | H | H | Me | $CR^6$ | N | $-NH-CH_2Ph$ |
| H | H | H | Me | $CR^6$ | N | 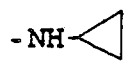 |
| H | H | H | Me | $CR^6$ | N | -NH-i-Pr |
| H | H | H | Me | $CR^6$ | N | 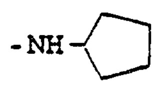 |
| H | H | H | Me | $CR^6$ | N | 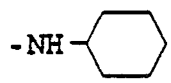 |
| H | H | H | Me | $CR^6$ | N | $-NMe_2$ |
| H | H | H | Me | $CR^6$ | N | $-NEt_2$ |
| H | H | H | Me | $CR^6$ | N | $-N(iPr)_2$ |
| H | H | H | Me | $CR^6$ | N | 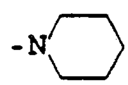 |
| H | H | H | Me | $CR^6$ | N | 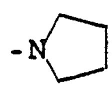 |
| H | H | H | Me | $CR^6$ | N | 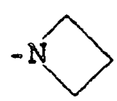 |
| H | H | H | Me | $CR^6$ | N | 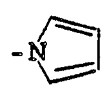 |
| H | H | H | Me | $CR^6$ | N | 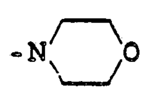 |
| H | H | H | Me | $CR^6$ | N | 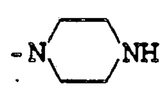 |

| $R^1$ | $R^2$ | $R^3$ | $R^6$ | X | Y | Z |
|---|---|---|---|---|---|---|
| Cl | H | H | Et | N | $CR^6$ | $-NH_2$ |
| Cl | H | H | Et | N | $CR^6$ | -NHMe |
| Cl | H | H | Et | N | $CR^6$ | -NHEt |
| H | H | H | Et | N | $CR^6$ | -NHPh |
| Cl | H | H | Et | N | $CR^6$ | $-NH-CH_2Ph$ |
| Cl | H | H | Et | N | $CR^6$ | 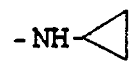 |
| Cl | H | H | Et | N | $CR^6$ | -NH-i-Pr |
| Cl | H | H | Et | N | $CR^6$ | 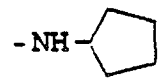 |
| Cl | H | H | Et | N | $CR^6$ | 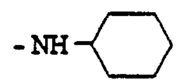 |
| Cl | H | H | Et | N | $CR^6$ | $-NMe_2$ |
| Cl | H | H | Et | N | $CR^6$ | $-NEt_2$ |
| Cl | H | H | Et | N | $CR^6$ | $-N(iPr)_2$ |
| Cl | H | H | Et | N | $CR^6$ | 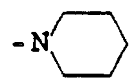 |
| Cl | H | H | Et | N | $CR^6$ | 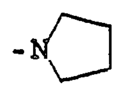 |
| Cl | H | H | Et | N | $CR^6$ | 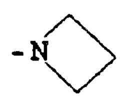 |
| Cl | H | H | Et | N | $CR^6$ | 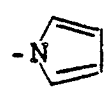 |
| Cl | H | H | Et | N | $CR^6$ | 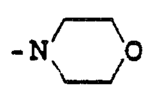 |
| Cl | H | H | Et | N | $CR^6$ | 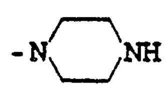 |

| $R^1$ | $R^2$ | $R^3$ | $R^6$ | X | Y | Z |
|---|---|---|---|---|---|---|
| Cl | H | H | Et | $CR^6$ | N | $-NH_2$ |
| Cl | H | H | Et | $CR^6$ | N | -NHMe |
| Cl | H | H | Et | $CR^6$ | N | -NHEt |
| Cl | H | H | Et | $CR^6$ | N | -NHPh |
| Cl | H | H | Et | $CR^6$ | N | $-NH-CH_2Ph$ |
| Cl | H | H | Et | $CR^6$ | N | 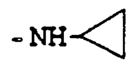 |
| Cl | H | H | Et | $CR^6$ | N | -NH-i-Pr |
| Cl | H | H | Et | $CR^6$ | N | 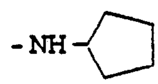 |
| Cl | H | H | Et | $CR^6$ | N | 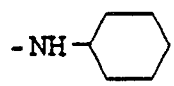 |
| Cl | H | H | Et | $CR^6$ | N | $-NMe_2$ |
| Cl | H | H | Et | $CR^6$ | N | $-NEt_2$ |
| Cl | H | H | Et | $CR^6$ | N | $-N(iPr)_2$ |
| Cl | H | H | Et | $CR^6$ | N | 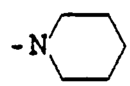 |
| Cl | H | H | Et | $CR^6$ | N | 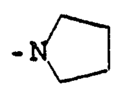 |
| Cl | H | H | Et | $CR^6$ | N | 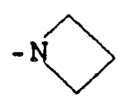 |
| Cl | H | H | Et | $CR^6$ | N | 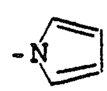 |
| Cl | H | H | Et | $CR^6$ | N | 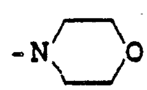 |
| Cl | H | H | Et | $CR^6$ | N | 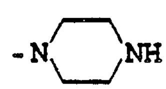 |

| $R^1$ | $R^2$ | $R^3$ | $R^6$ | X | Y | Z |
|---|---|---|---|---|---|---|
| Cl | H | H | Me | N | $CR^6$ | $-NH_2$ |
| Cl | H | H | Me | N | $CR^6$ | -NHMe |
| Cl | H | H | Me | N | $CR^6$ | -NHEt |
| Cl | H | H | Me | N | $CR^6$ | -NHPh |
| Cl | H | H | Me | N | $CR^6$ | $-NH-CH_2Ph$ |
| Cl | H | H | Me | N | $CR^6$ | -NH 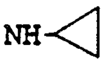 |
| Cl | H | H | Me | N | $CR^6$ | -NH-i-Pr |
| Cl | H | H | Me | N | $CR^6$ | -NH 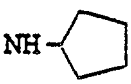 |
| Cl | H | H | Me | N | $CR^6$ | -NH 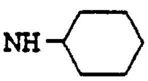 |
| Cl | H | H | Me | N | $CR^6$ | $-NMe_2$ |
| Cl | H | H | Me | N | $CR^6$ | $-NEt_2$ |
| Cl | H | H | Me | N | $CR^6$ | $-N(iPr)_2$ |
| Cl | H | H | Me | N | $CR^6$ | - 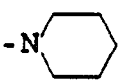 |
| Cl | H | H | Me | N | $CR^6$ | - 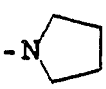 |
| Cl | H | H | Me | N | $CR^6$ | - 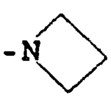 |
| Cl | H | H | Me | N | $CR^6$ | - 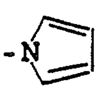 |
| Cl | H | H | Me | N | $CR^6$ | - 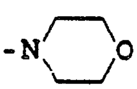 |
| Cl | H | H | Me | N | $CR^6$ | - 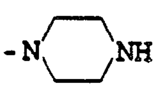 |

| $R^1$ | $R^2$ | $R^3$ | $R^6$ | X | Y | Z |
|---|---|---|---|---|---|---|
| Cl | H | H | Me | $CR^6$ | N | $-NH_2$ |
| Cl | H | H | Me | $CR^6$ | N | -NHMe |
| Cl | H | H | Me | $CR^6$ | N | -NHEt |
| Cl | H | H | Me | $CR^6$ | N | -NHPh |
| Cl | H | H | Me | $CR^6$ | N | $-NH-CH_2Ph$ |
| Cl | H | H | Me | $CR^6$ | N | -NH 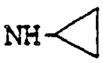 |
| Cl | H | H | Me | $CR^6$ | N | -NH-i-Pr |
| Cl | H | H | Me | $CR^6$ | N | -NH 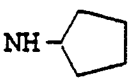 |
| Cl | H | H | Me | $CR^6$ | N | -NH 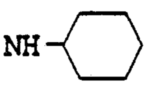 |
| Cl | H | H | Me | $CR^6$ | N | $-NMe_2$ |
| Cl | H | H | Me | $CR^6$ | N | $-NEt_2$ |
| Cl | H | H | Me | $CR^6$ | N | $-N(iPr)_2$ |
| Cl | H | H | Me | $CR^6$ | N | - 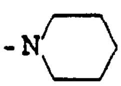 |
| Cl | H | H | Me | $CR^6$ | N | - 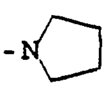 |
| Cl | H | H | Me | $CR^6$ | N | - 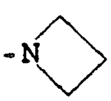 |
| Cl | H | H | Me | $CR^6$ | N | - 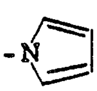 |
| Cl | H | H | Me | $CR^6$ | N | - 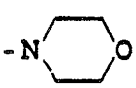 |
| Cl | H | H | Me | $CR^6$ | N | - 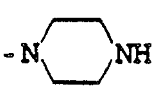 |

| $R^1$ | $R^2$ | $R^3$ | $R^6$ | X | Y | Z |
|---|---|---|---|---|---|---|
| H | H | $CO_2t$-Bu | i-Pr | $CR^6$ | N | $-NH_2$ |
| H | H | $CO_2t$-Bu | i-Pr | $CR^6$ | N | -NHMe |
| H | H | $CO_2t$-Bu | i-Pr | $CR^6$ | N | -NHEt |
| H | H | $CO_2t$-Bu | i-Pr | $CR^6$ | N | -NHPh |
| H | H | $CO_2t$-Bu | i-Pr | $CR^6$ | N | $-NH-CH_2Ph$ |
| H | H | $CO_2t$-Bu | i-Pr | $CR^6$ | N | -NH 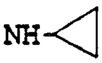 |
| H | H | $CO_2t$-Bu | i-Pr | $CR^6$ | N | -NH-i-Pr |
| H | H | $CO_2t$-Bu | i-Pr | $CR^6$ | N | -NH 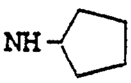 |
| H | H | $CO_2t$-Bu | i-Pr | $CR^6$ | N | -NH 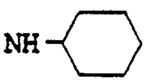 |
| H | H | $CO_2t$-Bu | i-Pr | $CR^6$ | N | $-NMe_2$ |
| H | H | $CO_2t$-Bu | i-Pr | $CR^6$ | N | $-NEt_2$ |
| H | H | $CO_2t$-Bu | i-Pr | $CR^6$ | N | $-N(iPr)_2$ |
| H | H | $CO_2t$-Bu | i-Pr | $CR^6$ | N | 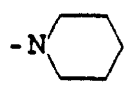 |
| H | H | $CO_2t$-Bu | i-Pr | $CR^6$ | N | 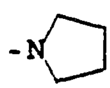 |
| H | H | $CO_2t$-Bu | i-Pr | $CR^6$ | N | 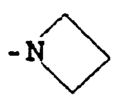 |
| H | H | $CO_2t$-Bu | i-Pr | $CR^6$ | N | 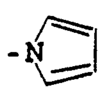 |
| H | H | $CO_2t$-Bu | i-Pr | $CR^6$ | N | 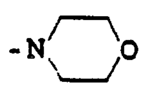 |
| H | H | $CO_2t$-Bu | i-Pr | $CR^6$ | N | 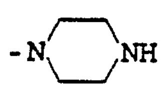 |

| $R^1$ | $R^2$ | $R^3$ | $R^6$ | X | Y | Z |
|---|---|---|---|---|---|---|
| H | H | $CO_2$t-Bu | i-Pr | N | $CR^6$ | $-NH_2$ |
| H | H | $CO_2$t-Bu | i-Pr | N | $CR^6$ | -NHMe |
| H | H | $CO_2$t-Bu | i-Pr | N | $CR^6$ | -NHEt |
| H | H | $CO_2$t-Bu | i-Pr | N | $CR^6$ | -NHPh |
| H | H | $CO_2$t-Bu | i-Pr | N | $CR^6$ | $-NH-CH_2Ph$ |
| H | H | $CO_2$t-Bu | i-Pr | N | $CR^6$ | -NH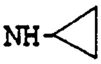 |
| H | H | $CO_2$t-Bu | i-Pr | N | $CR^6$ | -NH-i-Pr |
| H | H | $CO_2$t-Bu | i-Pr | N | $CR^6$ | -NH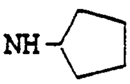 |
| H | H | $CO_2$t-Bu | i-Pr | N | $CR^6$ | -NH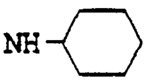 |
| H | H | $CO_2$t-Bu | i-Pr | N | $CR^6$ | $-NMe_2$ |
| H | H | $CO_2$t-Bu | i-Pr | N | $CR^6$ | $-NEt_2$ |
| H | H | $CO_2$t-Bu | i-Pr | N | $CR^6$ | $-N(iPr)_2$ |
| H | H | $CO_2$t-Bu | i-Pr | N | $CR^6$ | -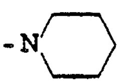 |
| H | H | $CO_2$t-Bu | i-Pr | N | $CR^6$ | -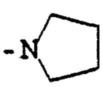 |
| H | H | $CO_2$t-Bu | i-Pr | N | $CR^6$ | -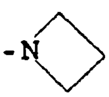 |
| H | H | $CO_2$t-Bu | i-Pr | N | $CR^6$ | -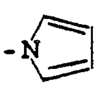 |
| H | H | $CO_2$t-Bu | i-Pr | N | $CR^6$ | -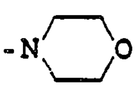 |
| H | H | $CO_2$t-Bu | i-Pr | N | $CR^6$ | -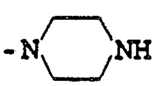 |

| $R^1$ | $R^2$ | $R^3$ | $R^6$ | X | Y | Z |
|---|---|---|---|---|---|---|
| H | H | $CO_2$t-Bu | Et | N | $CR^6$ | $-NH_2$ |
| H | H | $CO_2$t-Bu | Et | N | $CR^6$ | -NHMe |
| H | H | $CO_2$t-Bu | Et | N | $CR^6$ | -NHEt |
| H | H | $CO_2$t-Bu | Et | N | $CR^6$ | -NHPh |
| H | H | $CO_2$t-Bu | Et | N | $CR^6$ | $-NH-CH_2Ph$ |
| H | H | $CO_2$t-Bu | Et | N | $CR^6$ | -NH- 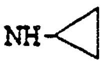 |
| H | H | $CO_2$t-Bu | Et | N | $CR^6$ | -NH-i-Pr |
| H | H | $CO_2$t-Bu | Et | N | $CR^6$ | -NH- 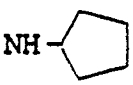 |
| H | H | $CO_2$t-Bu | Et | N | $CR^6$ | -NH- 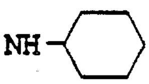 |
| H | H | $CO_2$t-Bu | Et | N | $CR^6$ | $-NMe_2$ |
| H | H | $CO_2$t-Bu | Et | N | $CR^6$ | $-NEt_2$ |
| H | H | $CO_2$t-Bu | Et | N | $CR^6$ | $-N(iPr)_2$ |
| H | H | $CO_2$t-Bu | Et | N | $CR^6$ | - 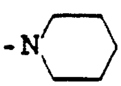 |
| H | H | $CO_2$t-Bu | Et | N | $CR^6$ | - 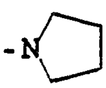 |
| H | H | $CO_2$t-Bu | Et | N | $CR^6$ | - 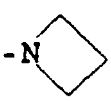 |
| H | H | $CO_2$t-Bu | Et | N | $CR^6$ | - 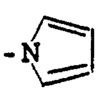 |
| H | H | $CO_2$t-Bu | Et | N | $CR^6$ | - 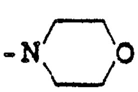 |
| H | H | $CO_2$t-Bu | Et | N | $CR^6$ | - 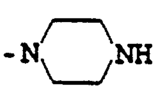 |

| $R^1$ | $R^2$ | $R^3$ | $R^6$ | X | Y | Z |
|---|---|---|---|---|---|---|
| H | H | $CO_2$t-Bu | Et | $CR^6$ | N | $-NH_2$ |
| H | H | $CO_2$t-Bu | Et | $CR^6$ | N | -NHMe |
| H | H | $CO_2$t-Bu | Et | $CR^6$ | N | -NHEt |
| H | H | $CO_2$t-Bu | Et | $CR^6$ | N | -NHPh |
| H | H | $CO_2$t-Bu | Et | $CR^6$ | N | $-NH-CH_2Ph$ |
| H | H | $CO_2$t-Bu | Et | $CR^6$ | N | -NH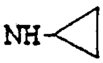 |
| H | H | $CO_2$t-Bu | Et | $CR^6$ | N | -NH-i-Pr |
| H | H | $CO_2$t-Bu | Et | $CR^6$ | N | -NH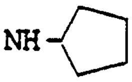 |
| H | H | $CO_2$t-Bu | Et | $CR^6$ | N | -NH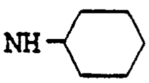 |
| H | H | $CO_2$t-Bu | Et | $CR^6$ | N | $-NMe_2$ |
| H | H | $CO_2$t-Bu | Et | $CR^6$ | N | $-NEt_2$ |
| H | H | $CO_2$t-Bu | Et | $CR^6$ | N | $-N(iPr)_2$ |
| H | H | $CO_2$t-Bu | Et | $CR^6$ | N | -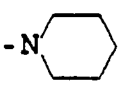 |
| H | H | $CO_2$t-Bu | Et | $CR^6$ | N | -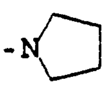 |
| H | H | $CO_2$t-Bu | Et | $CR^6$ | N | -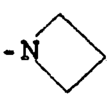 |
| H | H | $CO_2$t-Bu | Et | $CR^6$ | N | -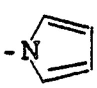 |
| H | H | $CO_2$t-Bu | Et | $CR^6$ | N | -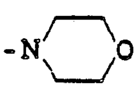 |
| H | H | $CO_2$t-Bu | Et | $CR^6$ | N | -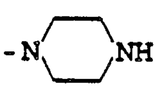 |

| $R^1$ | $R^2$ | $R^3$ | $R^6$ | X | Y | Z |
|---|---|---|---|---|---|---|
| H | H | $CO_2t$-Bu | $NH_2$ | N | $CR^6$ | $-NH_2$ |
| H | H | $CO_2t$-Bu | $NH_2$ | N | $CR^6$ | -NHMe |
| H | H | $CO_2t$-Bu | $NH_2$ | N | $CR^6$ | -NHEt |
| H | H | $CO_2t$-Bu | $NH_2$ | N | $CR^6$ | -NHPh |
| H | H | $CO_2t$-Bu | $NH_2$ | N | $CR^6$ | $-NH-CH_2Ph$ |
| H | H | $CO_2t$-Bu | $NH_2$ | N | $CR^6$ | -NH 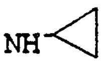 |
| H | H | $CO_2t$-Bu | $NH_2$ | N | $CR^6$ | -NH-i-Pr |
| H | H | $CO_2t$-Bu | $NH_2$ | N | $CR^6$ | -NH 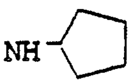 |
| H | H | $CO_2t$-Bu | $NH_2$ | N | $CR^6$ | -NH 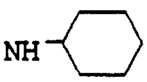 |
| H | H | $CO_2t$-Bu | $NH_2$ | N | $CR^6$ | $-NMe_2$ |
| H | H | $CO_2t$-Bu | $NH_2$ | N | $CR^6$ | $-NEt_2$ |
| H | H | $CO_2t$-Bu | $NH_2$ | N | $CR^6$ | $-N(iPr)_2$ |
| H | H | $CO_2t$-Bu | $NH_2$ | N | $CR^6$ | - 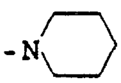 |
| H | H | $CO_2t$-Bu | $NH_2$ | N | $CR^6$ | - 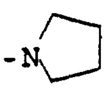 |
| H | H | $CO_2t$-Bu | $NH_2$ | N | $CR^6$ | - 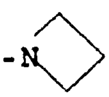 |
| H | H | $CO_2t$-Bu | $NH_2$ | N | $CR^6$ | - 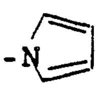 |
| H | H | $CO_2t$-Bu | $NH_2$ | N | $CR^6$ | - 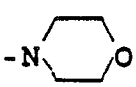 |
| H | H | $CO_2t$-Bu | $NH_2$ | N | $CR^6$ | - 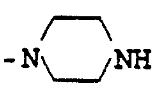 |

| $R^1$ | $R^2$ | $R^3$ | $R^6$ | X | Y | Z |
|---|---|---|---|---|---|---|
| H | H | $CO_2t\text{-}Bu$ | Me | N | $CR^6$ | $-NH_2$ |
| H | H | $CO_2t\text{-}Bu$ | Me | N | $CR^6$ | -NHMe |
| H | H | $CO_2t\text{-}Bu$ | Me | N | $CR^6$ | -NHEt |
| H | H | $CO_2t\text{-}Bu$ | Me | N | $CR^6$ | -NHPh |
| H | H | $CO_2t\text{-}Bu$ | Me | N | $CR^6$ | $-NH\text{-}CH_2Ph$ |
| H | H | $CO_2t\text{-}Bu$ | Me | N | $CR^6$ | -NH 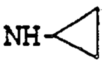 |
| H | H | $CO_2t\text{-}Bu$ | Me | N | $CR^6$ | -NH-i-Pr |
| H | H | $CO_2t\text{-}Bu$ | Me | N | $CR^6$ | -NH 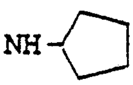 |
| H | H | $CO_2t\text{-}Bu$ | Me | N | $CR^6$ | -NH 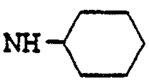 |
| H | H | $CO_2t\text{-}Bu$ | Me | N | $CR^6$ | $-NMe_2$ |
| H | H | $CO_2t\text{-}Bu$ | Me | N | $CR^6$ | $-NEt_2$ |
| H | H | $CO_2t\text{-}Bu$ | Me | N | $CR^6$ | $-N(iPr)_2$ |
| H | H | $CO_2t\text{-}Bu$ | Me | N | $CR^6$ | - 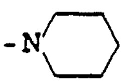 |
| H | H | $CO_2t\text{-}Bu$ | Me | N | $CR^6$ | - 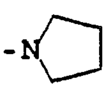 |
| H | H | $CO_2t\text{-}Bu$ | Me | N | $CR^6$ | - 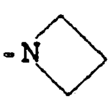 |
| H | H | $CO_2t\text{-}Bu$ | Me | N | $CR^6$ | - 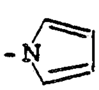 |
| H | H | $CO_2t\text{-}Bu$ | Me | N | $CR^6$ | - 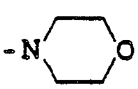 |
| H | H | $CO_2t\text{-}Bu$ | Me | N | $CR^6$ | - 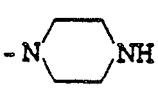 |

| $R^1$ | $R^2$ | $R^3$ | $R^6$ | X | Y | Z |
|---|---|---|---|---|---|---|
| H | H | $CO_2t$-Bu | Me | $CR^6$ | N | $-NH_2$ |
| H | H | $CO_2t$-Bu | Me | $CR^6$ | N | -NHMe |
| H | H | $CO_2t$-Bu | Me | $CR^6$ | N | -NHEt |
| H | H | $CO_2t$-Bu | Me | $CR^6$ | N | -NHPh |
| H | H | $CO_2t$-Bu | Me | $CR^6$ | N | $-NH-CH_2Ph$ |
| H | H | $CO_2t$-Bu | Me | $CR^6$ | N | -NH- 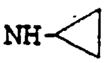 |
| H | H | $CO_2t$-Bu | Me | $CR^6$ | N | -NH-i-Pr |
| H | H | $CO_2t$-Bu | Me | $CR^6$ | N | -NH- 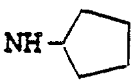 |
| H | H | $CO_2t$-Bu | Me | $CR^6$ | N | -NH- 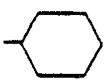 |
| H | H | $CO_2t$-Bu | Me | $CR^6$ | N | $-NMe_2$ |
| H | H | $CO_2t$-Bu | Me | $CR^6$ | N | $-NEt_2$ |
| H | H | $CO_2t$-Bu | Me | $CR^6$ | N | $-N(iPr)_2$ |
| H | H | $CO_2t$-Bu | Me | $CR^6$ | N | 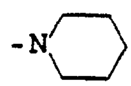 |
| H | H | $CO_2t$-Bu | Me | $CR^6$ | N | 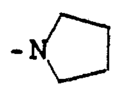 |
| H | H | $CO_2t$-Bu | Me | $CR^6$ | N | 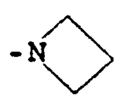 |
| H | H | $CO_2t$-Bu | Me | $CR^6$ | N | 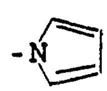 |
| H | H | $CO_2t$-Bu | Me | $CR^6$ | N | 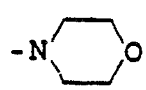 |
| H | H | $CO_2t$-Bu | Me | $CR^6$ | N | 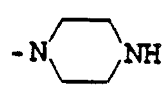 |

| $R^1$ | $R^2$ | $R^3$ | $R^6$ | X | Y | Z |
|---|---|---|---|---|---|---|
| H | H | H | $NH_2$ | $CR^6$ | N | $-NH_2$ |
| H | H | H | $NH_2$ | $CR^6$ | N | -NHMe |
| H | H | H | $NH_2$ | $CR^6$ | N | -NHEt |
| H | H | H | $NH_2$ | $CR^6$ | N | -NHPh |
| H | H | H | $NH_2$ | $CR^6$ | N | $-NH-CH_2Ph$ |
| H | H | H | $NH_2$ | $CR^6$ | N | -NH 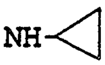 |
| H | H | H | $NH_2$ | $CR^6$ | N | -NH-i-Pr |
| H | H | H | $NH_2$ | $CR^6$ | N | -NH 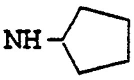 |
| H | H | H | $NH_2$ | $CR^6$ | N | -NH 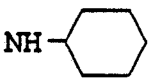 |
| H | H | H | $NH_2$ | $CR^6$ | N | $-NMe_2$ |
| H | H | H | $NH_2$ | $CR^6$ | N | $-NEt_2$ |
| H | H | H | $NH_2$ | $CR^6$ | N | $-N(iPr)_2$ |
| H | H | H | $NH_2$ | $CR^6$ | N | - 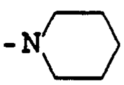 |
| H | H | H | $NH_2$ | $CR^6$ | N | - 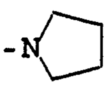 |
| H | H | H | $NH_2$ | $CR^6$ | N | - 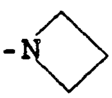 |
| H | H | H | $NH_2$ | $CR^6$ | N | - 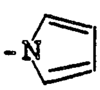 |
| H | H | H | $NH_2$ | $CR^6$ | N | - 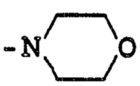 |
| H | H | H | $NH_2$ | $CR^6$ | N | - 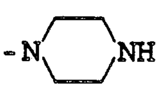 |

| $R^1$ | $R^2$ | $R^3$ | $R^6$ | X | Y | Z |
|---|---|---|---|---|---|---|
| H | H | H | $NH_2$ | N | $CR^6$ | $-NH_2$ |
| H | H | H | $NH_2$ | N | $CR^6$ | -NHMe |
| H | H | H | $NH_2$ | N | $CR^6$ | -NHEt |
| H | H | H | $NH_2$ | N | $CR^6$ | -NHPh |
| H | H | H | $NH_2$ | N | $CR^6$ | $-NH-CH_2Ph$ |
| H | H | H | $NH_2$ | N | $CR^6$ | 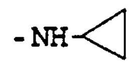 |
| H | H | H | $NH_2$ | N | $CR^6$ | -NH-i-Pr |
| H | H | H | $NH_2$ | N | $CR^6$ | 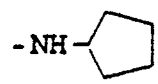 |
| H | H | H | $NH_2$ | N | $CR^6$ | 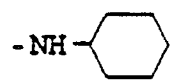 |
| H | H | H | $NH_2$ | N | $CR^6$ | $-NMe_2$ |
| H | H | H | $NH_2$ | N | $CR^6$ | $-NEt_2$ |
| H | H | H | $NH_2$ | N | $CR^6$ | $-N(iPr)_2$ |
| H | H | H | $NH_2$ | N | $CR^6$ | 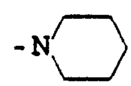 |
| H | H | H | $NH_2$ | N | $CR^6$ | 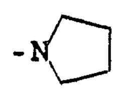 |
| H | H | H | $NH_2$ | N | $CR^6$ | 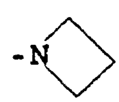 |
| H | H | H | $NH_2$ | N | $CR^6$ | 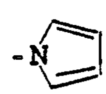 |
| H | H | H | $NH_2$ | N | $CR^6$ | 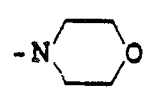 |
| H | H | H | $NH_2$ | N | $CR^6$ | 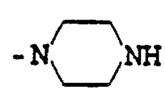 |

| $R^1$ | $R^2$ | $R^3$ | $R^6$ | X | Y | Z |
|---|---|---|---|---|---|---|
| H | H | $CO_2$t-Bu | $NH_2$ | $CR^6$ | N | $-NH_2$ |
| H | H | $CO_2$t-Bu | $NH_2$ | $CR^6$ | N | -NHMe |
| H | H | $CO_2$t-Bu | $NH_2$ | $CR^6$ | N | -NHEt |
| H | H | $CO_2$t-Bu | $NH_2$ | $CR^6$ | N | -NHPh |
| H | H | $CO_2$t-Bu | $NH_2$ | $CR^6$ | N | $-NH-CH_2Ph$ |
| H | H | $CO_2$t-Bu | $NH_2$ | $CR^6$ | N | -NH 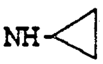 |
| H | H | $CO_2$t-Bu | $NH_2$ | $CR^6$ | N | -NH-i-Pr |
| H | H | $CO_2$t-Bu | $NH_2$ | $CR^6$ | N | -NH 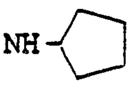 |
| H | H | $CO_2$t-Bu | $NH_2$ | $CR^6$ | N | -NH 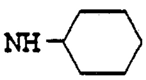 |
| H | H | $CO_2$t-Bu | $NH_2$ | $CR^6$ | N | $-NMe_2$ |
| H | H | $CO_2$t-Bu | $NH_2$ | $CR^6$ | N | $-NEt_2$ |
| H | H | $CO_2$t-Bu | $NH_2$ | $CR^6$ | N | $-N(iPr)_2$ |
| H | H | $CO_2$t-Bu | $NH_2$ | $CR^6$ | N | - 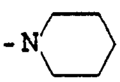 |
| H | H | $CO_2$t-Bu | $NH_2$ | $CR^6$ | N | - 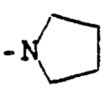 |
| H | H | $CO_2$t-Bu | $NH_2$ | $CR^6$ | N | - 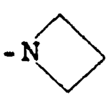 |
| H | H | $CO_2$t-Bu | $NH_2$ | $CR^6$ | N | - 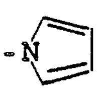 |
| H | H | $CO_2$t-Bu | $NH_2$ | $CR^6$ | N | - 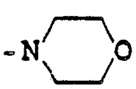 |
| H | H | $CO_2$t-Bu | $NH_2$ | $CR^6$ | N | - 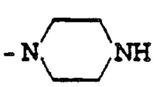 |

| $R^1$ | $R^2$ | $R^3$ | $R^6$ | X | Y | Z |
|---|---|---|---|---|---|---|
| Cl | H | H | Cl | $CR^6$ | N | $-NH_2$ |
| Cl | H | H | Cl | $CR^6$ | N | -NHMe |
| Cl | H | H | Cl | $CR^6$ | N | -NHEt |
| Cl | H | H | Cl | $CR^6$ | N | -NHPh |
| Cl | H | H | Cl | $CR^6$ | N | $-NH-CH_2Ph$ |
| Cl | H | H | Cl | $CR^6$ | N | -NH- 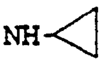 |
| Cl | H | H | Cl | $CR^6$ | N | -NH-i-Pr |
| Cl | H | H | Cl | $CR^6$ | N | -NH- 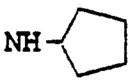 |
| Cl | H | H | Cl | $CR^6$ | N | -NH- 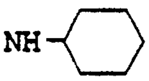 |
| Cl | H | H | Cl | $CR^6$ | N | $-NMe_2$ |
| Cl | H | H | Cl | $CR^6$ | N | $-NEt_2$ |
| Cl | H | H | Cl | $CR^6$ | N | $-N(iPr)_2$ |
| Cl | H | H | Cl | $CR^6$ | N | - 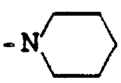 |
| Cl | H | H | Cl | $CR^6$ | N | - 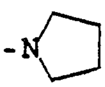 |
| Cl | H | H | Cl | $CR^6$ | N | - 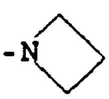 |
| Cl | H | H | Cl | $CR^6$ | N | - 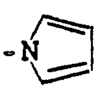 |
| Cl | H | H | Cl | $CR^6$ | N | - 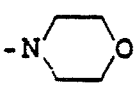 |
| Cl | H | H | Cl | $CR^6$ | N | - 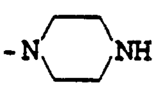 |

| $R^1$ | $R^2$ | $R^3$ | $R^6$ | X | Y | Z |
|---|---|---|---|---|---|---|
| Cl | H | H | Cl | N | $CR^6$ | $-NH_2$ |
| Cl | H | H | Cl | N | $CR^6$ | -NHMe |
| Cl | H | H | Cl | N | $CR^6$ | -NHEt |
| Cl | H | H | Cl | N | $CR^6$ | -NHPh |
| Cl | H | H | Cl | N | $CR^6$ | $-NH-CH_2Ph$ |
| Cl | H | H | Cl | N | $CR^6$ | -NH- 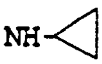 |
| Cl | H | H | Cl | N | $CR^6$ | -NH-i-Pr |
| Cl | H | H | Cl | N | $CR^6$ | -NH- 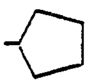 |
| Cl | H | H | Cl | N | $CR^6$ | -NH- 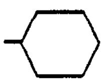 |
| Cl | H | H | Cl | N | $CR^6$ | $-NMe_2$ |
| Cl | H | H | Cl | N | $CR^6$ | $-NEt_2$ |
| Cl | H | H | Cl | N | $CR^6$ | $-N(iPr)_2$ |
| Cl | H | H | Cl | N | $CR^6$ | - 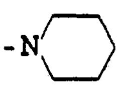 |
| Cl | H | H | Cl | N | $CR^6$ | - 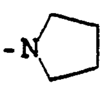 |
| Cl | H | H | Cl | N | $CR^6$ | - 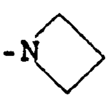 |
| Cl | H | H | Cl | N | $CR^6$ | - 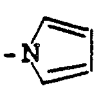 |
| Cl | H | H | Cl | N | $CR^6$ | - 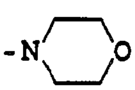 |
| Cl | H | H | Cl | N | $CR^6$ | - 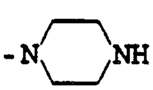 |

| $R^1$ | $R^2$ | $R^3$ | $R^6$ | X | Y | Z |
|---|---|---|---|---|---|---|
| Cl | H | H | i-Pr | N | $CR^6$ | $-NH_2$ |
| Cl | H | H | i-Pr | N | $CR^6$ | -NHMe |
| Cl | H | H | i-Pr | N | $CR^6$ | -NHEt |
| Cl | H | H | i-Pr | N | $CR^6$ | -NHPh |
| Cl | H | H | i-Pr | N | $CR^6$ | $-NH-CH_2Ph$ |
| Cl | H | H | i-Pr | N | $CR^6$ | -NH 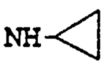 |
| Cl | H | H | i-Pr | N | $CR^6$ | -NH-i-Pr |
| Cl | H | H | i-Pr | N | $CR^6$ | -NH 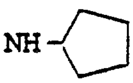 |
| Cl | H | H | i-Pr | N | $CR^6$ | -NH 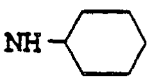 |
| Cl | H | H | i-Pr | N | $CR^6$ | $-NMe_2$ |
| Cl | H | H | i-Pr | N | $CR^6$ | $-NEt_2$ |
| Cl | H | H | i-Pr | N | $CR^6$ | $-N(iPr)_2$ |
| Cl | H | H | i-Pr | N | $CR^6$ | - 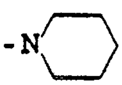 |
| Cl | H | H | i-Pr | N | $CR^6$ | - 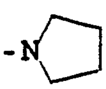 |
| Cl | H | H | i-Pr | N | $CR^6$ | - 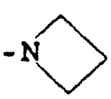 |
| Cl | H | H | i-Pr | N | $CR^6$ | - 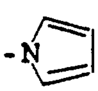 |
| Cl | H | H | i-Pr | N | $CR^6$ | - 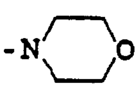 |
| Cl | H | H | i-Pr | N | $CR^6$ | - 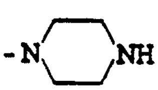 |

| $R^1$ | $R^2$ | $R^3$ | $R^6$ | X | Y | Z |
|---|---|---|---|---|---|---|
| Cl | H | H | i-Pr | $CR^6$ | N | $-NH_2$ |
| Cl | H | H | i-Pr | $CR^6$ | N | -NHMe |
| Cl | H | H | i-Pr | $CR^6$ | N | -NHEt |
| Cl | H | H | i-Pr | $CR^6$ | N | -NHPh |
| Cl | H | H | i-Pr | $CR^6$ | N | $-NH-CH_2Ph$ |
| Cl | H | H | i-Pr | $CR^6$ | N | -NH 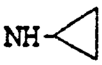 |
| Cl | H | H | i-Pr | $CR^6$ | N | -NH-i-Pr |
| Cl | H | H | i-Pr | $CR^6$ | N | -NH 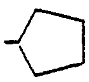 |
| Cl | H | H | i-Pr | $CR^6$ | N | -NH 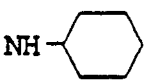 |
| Cl | H | H | i-Pr | $CR^6$ | N | $-NMe_2$ |
| Cl | H | H | i-Pr | $CR^6$ | N | $-NEt_2$ |
| Cl | H | H | i-Pr | $CR^6$ | N | $-N(iPr)_2$ |
| Cl | H | H | i-Pr | $CR^6$ | N | - 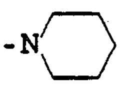 |
| Cl | H | H | i-Pr | $CR^6$ | N | - 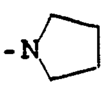 |
| Cl | H | H | i-Pr | $CR^6$ | N | - 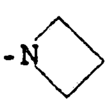 |
| Cl | H | H | i-Pr | $CR^6$ | N | - 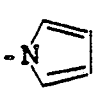 |
| Cl | H | H | i-Pr | $CR^6$ | N | - 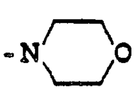 |
| Cl | H | H | i-Pr | $CR^6$ | N | - 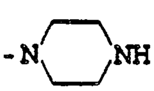 |

| $R^1$ | $R^2$ | $R^3$ | $R^6$ | X | Y | Z |
|---|---|---|---|---|---|---|
| H | H | $CO_2t$-Bu | Cl | N | $CR^6$ | $-NH_2$ |
| H | H | $CO_2t$-Bu | Cl | N | $CR^6$ | -NHMe |
| H | H | $CO_2t$-Bu | Cl | N | $CR^6$ | -NHEt |
| H | H | $CO_2t$-Bu | Cl | N | $CR^6$ | -NHPh |
| H | H | $CO_2t$-Bu | Cl | N | $CR^6$ | $-NH-CH_2Ph$ |
| H | H | $CO_2t$-Bu | Cl | N | $CR^6$ | -NH 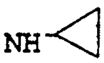 |
| H | H | $CO_2t$-Bu | Cl | N | $CR^6$ | -NH-i-Pr |
| H | H | $CO_2t$-Bu | Cl | N | $CR^6$ | -NH 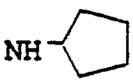 |
| H | H | $CO_2t$-Bu | Cl | N | $CR^6$ | -NH 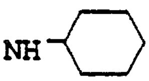 |
| H | H | $CO_2t$-Bu | Cl | N | $CR^6$ | $-NMe_2$ |
| H | H | $CO_2t$-Bu | Cl | N | $CR^6$ | $-NEt_2$ |
| H | H | $CO_2t$-Bu | Cl | N | $CR^6$ | $-N(iPr)_2$ |
| H | H | $CO_2t$-Bu | Cl | N | $CR^6$ | 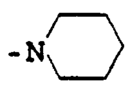 |
| H | H | $CO_2t$-Bu | Cl | N | $CR^6$ | 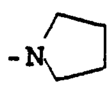 |
| H | H | $CO_2t$-Bu | Cl | N | $CR^6$ | 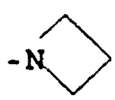 |
| H | H | $CO_2t$-Bu | Cl | N | $CR^6$ | 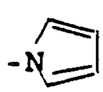 |
| H | H | $CO_2t$-Bu | Cl | N | $CR^6$ | 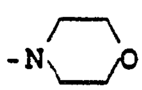 |
| H | H | $CO_2t$-Bu | Cl | N | $CR^6$ | 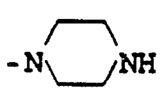 |

| $R^1$ | $R^2$ | $R^3$ | $R^6$ | X | Y | Z |
|---|---|---|---|---|---|---|
| H | H | $CO_2t$-Bu | Cl | $CR^6$ | N | $-NH_2$ |
| H | H | $CO_2t$-Bu | Cl | $CR^6$ | N | -NHMe |
| H | H | $CO_2t$-Bu | Cl | $CR^6$ | N | -NHEt |
| H | H | $CO_2t$-Bu | Cl | $CR^6$ | N | -NHPh |
| H | H | $CO_2t$-Bu | Cl | $CR^6$ | N | $-NH-CH_2Ph$ |
| H | H | $CO_2t$-Bu | Cl | $CR^6$ | N | -NH 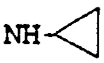 |
| H | H | $CO_2t$-Bu | Cl | $CR^6$ | N | -NH-i-Pr |
| H | H | $CO_2t$-Bu | Cl | $CR^6$ | N | -NH 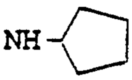 |
| H | H | $CO_2t$-Bu | Cl | $CR^6$ | N | -NH 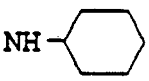 |
| H | H | $CO_2t$-Bu | Cl | $CR^6$ | N | $-NMe_2$ |
| H | H | $CO_2t$-Bu | Cl | $CR^6$ | N | $-NEt_2$ |
| H | H | $CO_2t$-Bu | Cl | $CR^6$ | N | $-N(iPr)_2$ |
| H | H | $CO_2t$-Bu | Cl | $CR^6$ | N | - 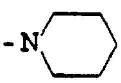 |
| H | H | $CO_2t$-Bu | Cl | $CR^6$ | N | - 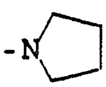 |
| H | H | $CO_2t$-Bu | Cl | $CR^6$ | N | - 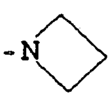 |
| H | H | $CO_2t$-Bu | Cl | $CR^6$ | N | - 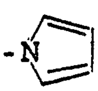 |
| H | H | $CO_2t$-Bu | Cl | $CR^6$ | N | - 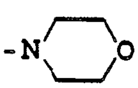 |
| H | H | $CO_2t$-Bu | Cl | $CR^6$ | N | - 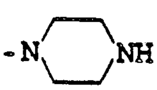 |

| $R^1$ | $R^2$ | $R^3$ | $R^6$ | X | Y | Z |
|---|---|---|---|---|---|---|
| Me | Me | H | H | N | $CR^6$ | $-NH_2$ |
| Me | Me | H | H | N | $CR^6$ | -NHMe |
| Me | Me | H | H | N | $CR^6$ | -NHEt |
| Me | Me | H | H | N | $CR^6$ | -NHPh |
| Me | Me | H | H | N | $CR^6$ | $-NH-CH_2Ph$ |
| Me | Me | H | H | N | $CR^6$ | -NH 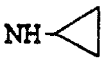 |
| Me | Me | H | H | N | $CR^6$ | -NH-i-Pr |
| Me | Me | H | H | N | $CR^6$ | -NH 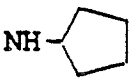 |
| Me | Me | H | H | N | $CR^6$ | -NH 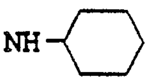 |
| Me | Me | H | H | N | $CR^6$ | $-NMe_2$ |
| Me | Me | H | H | N | $CR^6$ | $-NEt_2$ |
| Me | Me | H | H | N | $CR^6$ | $-N(iPr)_2$ |
| Me | Me | H | H | N | $CR^6$ | -N 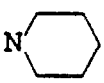 |
| Me | Me | H | H | N | $CR^6$ | -N 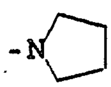 |
| Me | Me | H | H | N | $CR^6$ | -N 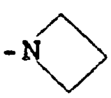 |
| Me | Me | H | H | N | $CR^6$ | -N 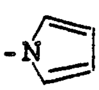 |
| Me | Me | H | H | N | $CR^6$ | -N 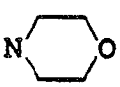 |
| Me | Me | H | H | N | $CR^6$ | -N 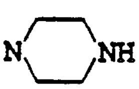 |

| $R^1$ | $R^2$ | $R^3$ | $R^6$ | X | Y | Z |
|---|---|---|---|---|---|---|
| Me | Me | H | H | $CR^6$ | N | $-NH_2$ |
| Me | Me | H | H | $CR^6$ | N | -NHMe |
| Me | Me | H | H | $CR^6$ | N | -NHEt |
| Me | Me | H | H | $CR^6$ | N | -NHPh |
| Me | Me | H | H | $CR^6$ | N | $-NH-CH_2Ph$ |
| Me | Me | H | H | $CR^6$ | N | -NH- 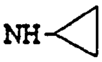 |
| Me | Me | H | H | $CR^6$ | N | -NH-i-Pr |
| Me | Me | H | H | $CR^6$ | N | -NH- 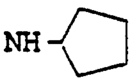 |
| Me | Me | H | H | $CR^6$ | N | -NH- 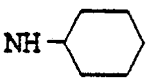 |
| Me | Me | H | H | $CR^6$ | N | $-NMe_2$ |
| Me | Me | H | H | $CR^6$ | N | $-NEt_2$ |
| Me | Me | H | H | $CR^6$ | N | $-N(iPr)_2$ |
| Me | Me | H | H | $CR^6$ | N | - 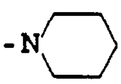 |
| Me | Me | H | H | $CR^6$ | N | - 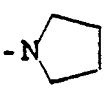 |
| Me | Me | H | H | $CR^6$ | N | - 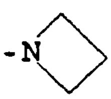 |
| Me | Me | H | H | $CR^6$ | N | - 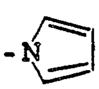 |
| Me | Me | H | H | $CR^6$ | N | - 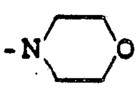 |
| Me | Me | H | H | $CR^6$ | N | - 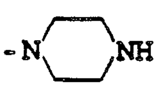 |

| $R^1$ | $R^2$ | $R^3$ | $R^6$ | X | Y | Z |
|---|---|---|---|---|---|---|
| H | H | H | 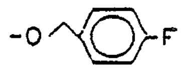 | N | $CR^6$ | $-NH_2$ |
| H | H | H | " | N | $CR^6$ | -NHMe |
| H | H | H | " | N | $CR^6$ | -NHEt |
| H | H | H | " | N | $CR^6$ | -NHPh |
| H | H | H | " | N | $CR^6$ | $-NH-CH_2Ph$ |
| H | H | H | " | N | $CR^6$ | -NH 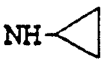 |
| H | H | H | " | N | $CR^6$ | -NH-i-Pr |
| H | H | H | " | N | $CR^6$ | -NH 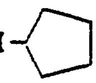 |
| H | H | H | " | N | $CR^6$ | -NH 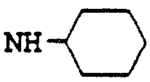 |
| H | H | H | " | N | $CR^6$ | $-NMe_2$ |
| H | H | H | " | N | $CR^6$ | $-NEt_2$ |
| H | H | H | " | N | $CR^6$ | $-N(iPr)_2$ |
| H | H | H | " | N | $CR^6$ | 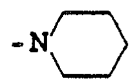 |
| H | H | H | " | N | $CR^6$ | 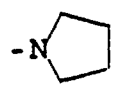 |
| H | H | H | " | N | $CR^6$ | 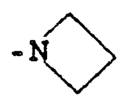 |
| H | H | H | " | N | $CR^6$ | 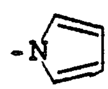 |
| H | H | H | " | N | $CR^6$ | 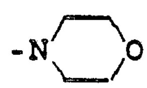 |
| H | H | H | " | N | $CR^6$ | 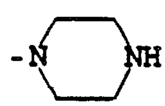 |

| $R^1$ | $R^2$ | $R^3$ | $R^6$ | X | Y | Z |
|---|---|---|---|---|---|---|
| H | H | H | 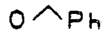 | N | $CR^6$ | $-NH_2$ |
| H | H | H | " | N | $CR^6$ | -NHMe |
| H | H | H | " | N | $CR^6$ | -NHEt |
| H | H | H | " | N | $CR^6$ | -NHPh |
| H | H | H | " | N | $CR^6$ | $-NH-CH_2Ph$ |
| H | H | H | " | N | $CR^6$ | 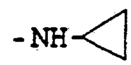 |
| H | H | H | " | N | $CR^6$ | -NH-i-Pr |
| H | H | H | " | N | $CR^6$ | 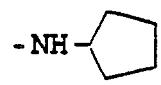 |
| H | H | H | " | N | $CR^6$ | 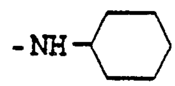 |
| H | H | H | " | N | $CR^6$ | $-NMe_2$ |
| H | H | H | " | N | $CR^6$ | $-NEt_2$ |
| H | H | H | " | N | $CR^6$ | $-N(iPr)_2$ |
| H | H | H | " | N | $CR^6$ | 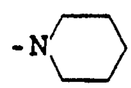 |
| H | H | H | " | N | $CR^6$ | 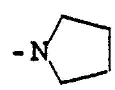 |
| H | H | H | " | N | $CR^6$ | 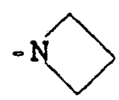 |
| H | H | H | " | N | $CR^6$ | 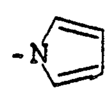 |
| H | H | H | " | N | $CR^6$ | 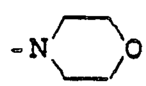 |
| H | H | H | " | N | $CR^6$ | 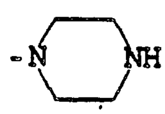 |

| $R^1$ | $R^2$ | $R^3$ | $R^6$ | X | Y | Z |
|---|---|---|---|---|---|---|
| H | H | H | 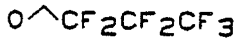 | N | $CR^6$ | $-NH_2$ |
| H | H | H | " | N | $CR^6$ | -NHMe |
| H | H | H | " | N | $CR^6$ | -NHEt |
| H | H | H | " | N | $CR^6$ | -NHPh |
| H | H | H | " | N | $CR^6$ | $-NH-CH_2Ph$ |
| H | H | H | " | N | $CR^6$ | -NH 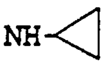 |
| H | H | H | " | N | $CR^6$ | -NH-i-Pr |
| H | H | H | " | N | $CR^6$ | -NH 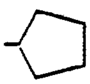 |
| H | H | H | " | N | $CR^6$ | -NH 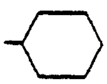 |
| H | H | H | " | N | $CR^6$ | $-NMe_2$ |
| H | H | H | " | N | $CR^6$ | $-NEt_2$ |
| H | H | H | " | N | $CR^6$ | $-N(iPr)_2$ |
| H | H | H | " | N | $CR^6$ | -N 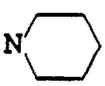 |
| H | H | H | " | N | $CR^6$ | -N 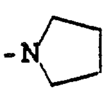 |
| H | H | H | " | N | $CR^6$ | -N 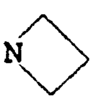 |
| H | H | H | " | N | $CR^6$ | -N 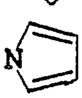 |
| H | H | H | " | N | $CR^6$ | -N 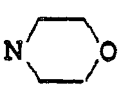 |
| H | H | H | " | N | $CR^6$ | -N 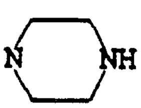 |

| $R^1$ | $R^2$ | $R^3$ | $R^6$ | X | Y | Z |
|---|---|---|---|---|---|---|
| H | H | H | 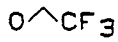 | N | $CR^6$ | $-NH_2$ |
| H | H | H | " | N | $CR^6$ | -NHMe |
| H | H | H | " | N | $CR^6$ | -NHEt |
| H | H | H | " | N | $CR^6$ | -NHPh |
| H | H | H | " | N | $CR^6$ | $-NH-CH_2Ph$ |
| H | H | H | " | N | $CR^6$ | -NH- 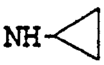 |
| H | H | H | " | N | $CR^6$ | -NH-i-Pr |
| H | H | H | " | N | $CR^6$ | -NH- 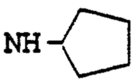 |
| H | H | H | " | N | $CR^6$ | -NH- 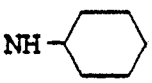 |
| H | H | H | " | N | $CR^6$ | $-NMe_2$ |
| H | H | H | " | N | $CR^6$ | $-NEt_2$ |
| H | H | H | " | N | $CR^6$ | $-N(iPr)_2$ |
| H | H | H | " | N | $CR^6$ | - 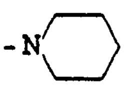 |
| H | H | H | " | N | $CR^6$ | - 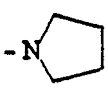 |
| H | H | H | " | N | $CR^6$ | - 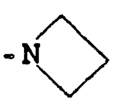 |
| H | H | H | " | N | $CR^6$ | - 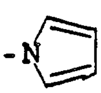 |
| H | H | H | " | N | $CR^6$ | - 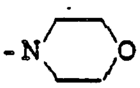 |
| H | H | H | " | N | $CR^6$ | - 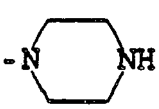 |

| $R^1$ | $R^2$ | $R^3$ | $R^6$ | X | Y | Z |
|---|---|---|---|---|---|---|
| H | H | H | 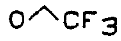 | $CR^6$ | N | $-NH_2$ |
| H | H | H | " | $CR^6$ | N | -NHMe |
| H | H | H | " | $CR^6$ | N | -NHEt |
| H | H | H | " | $CR^6$ | N | -NHPh |
| H | H | H | " | $CR^6$ | N | $-NH-CH_2Ph$ |
| H | H | H | " | $CR^6$ | N | 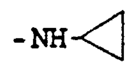 |
| H | H | H | " | $CR^6$ | N | -NH-i-Pr |
| H | H | H | " | $CR^6$ | N | 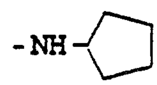 |
| H | H | H | " | $CR^6$ | N | 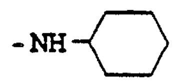 |
| H | H | H | " | $CR^6$ | N | $-NMe_2$ |
| H | H | H | " | $CR^6$ | N | $-NEt_2$ |
| H | H | H | " | $CR^6$ | N | $-N(iPr)_2$ |
| H | H | H | " | $CR^6$ | N | 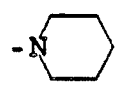 |
| H | H | H | " | $CR^6$ | N | 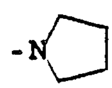 |
| H | H | H | " | $CR^6$ | N | 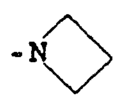 |
| H | H | H | " | $CR^6$ | N | 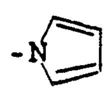 |
| H | H | H | " | $CR^6$ | N | 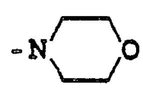 |
| H | H | H | " | $CR^6$ | N | 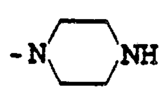 |

| $R^1$ | $R^2$ | $R^3$ | $R^6$ | X | Y | Z |
|---|---|---|---|---|---|---|
| H | H | H | 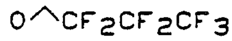 | $CR^6$ | N | $-NH_2$ |
| H | H | H | " | $CR^6$ | N | -NHMe |
| H | H | H | " | $CR^6$ | N | -NHEt |
| H | H | H | " | $CR^6$ | N | -NHPh |
| H | H | H | " | $CR^6$ | N | $-NH-CH_2Ph$ |
| H | H | H | " | $CR^6$ | N | -NH 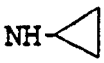 |
| H | H | H | " | $CR^6$ | N | -NH-i-Pr |
| H | H | H | " | $CR^6$ | N | -NH 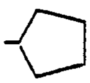 |
| H | H | H | " | $CR^6$ | N | -NH 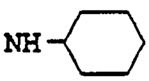 |
| H | H | H | " | $CR^6$ | N | $-NMe_2$ |
| H | H | H | " | $CR^6$ | N | $-NEt_2$ |
| H | H | H | " | $CR^6$ | N | $-N(iPr)_2$ |
| H | H | H | " | $CR^6$ | N | - 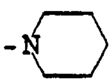 |
| H | H | H | " | $CR^6$ | N | - 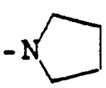 |
| H | H | H | " | $CR^6$ | N | - 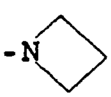 |
| H | H | H | " | $CR^6$ | N | - 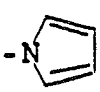 |
| H | H | H | " | $CR^6$ | N | - 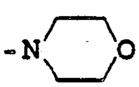 |
| H | H | H | " | $CR^6$ | N | - 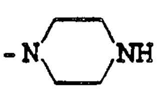 |

| $R^1$ | $R^2$ | $R^3$ | $R^6$ | X | Y | Z |
|---|---|---|---|---|---|---|
| H | H | H | 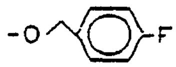 | $CR^6$ | N | $-NH_2$ |
| H | H | H | " | $CR^6$ | N | -NHMe |
| H | H | H | " | $CR^6$ | N | -NHEt |
| H | H | H | " | $CR^6$ | N | -NHPh |
| H | H | H | " | $CR^6$ | N | $-NH-CH_2Ph$ |
| H | H | H | " | $CR^6$ | N | -NH 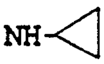 |
| H | H | H | " | $CR^6$ | N | -NH-i-Pr |
| H | H | H | " | $CR^6$ | N | -NH 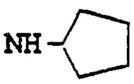 |
| H | H | H | " | $CR^6$ | N | -NH 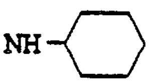 |
| H | H | H | " | $CR^6$ | N | $-NMe_2$ |
| H | H | H | " | $CR^6$ | N | $-NEt_2$ |
| H | H | H | " | $CR^6$ | N | $-N(iPr)_2$ |
| H | H | H | " | $CR^6$ | N | - 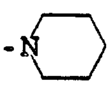 |
| H | H | H | " | $CR^6$ | N | - 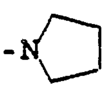 |
| H | H | H | " | $CR^6$ | N | - 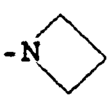 |
| H | H | H | " | $CR^6$ | N | - 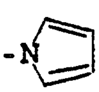 |
| H | H | H | " | $CR^6$ | N | - 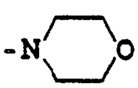 |
| H | H | H | " | $CR^6$ | N | - 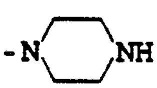 |

| $R^1$ | $R^2$ | $R^3$ | $R^6$ | X | Y | Z |
|---|---|---|---|---|---|---|
| H | H | H | 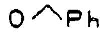 | $CR^6$ | N | $-NH_2$ |
| H | H | H | " | $CR^6$ | N | -NHMe |
| H | H | H | " | $CR^6$ | N | -NHEt |
| H | H | H | " | $CR^6$ | N | -NHPh |
| H | H | H | " | $CR^6$ | N | $-NH-CH_2Ph$ |
| H | H | H | " | $CR^6$ | N | -NH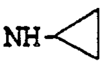 |
| H | H | H | " | $CR^6$ | N | -NH-i-Pr |
| H | H | H | " | $CR^6$ | N | -NH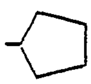 |
| H | H | H | " | $CR^6$ | N | -NH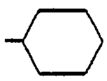 |
| H | H | H | " | $CR^6$ | N | $-NMe_2$ |
| H | H | H | " | $CR^6$ | N | $-NEt_2$ |
| H | H | H | " | $CR^6$ | N | $-N(iPr)_2$ |
| H | H | H | " | $CR^6$ | N | -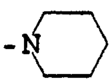 |
| H | H | H | " | $CR^6$ | N | -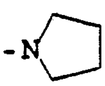 |
| H | H | H | " | $CR^6$ | N | -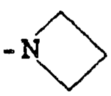 |
| H | H | H | " | $CR^6$ | N | -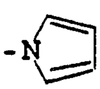 |
| H | H | H | " | $CR^6$ | N | -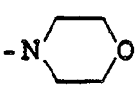 |
| H | H | H | " | $CR^6$ | N | -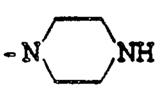 |

| $R^1$ | $R^2$ | $R^3$ | $R^6$ | X | Y | Z |
|---|---|---|---|---|---|---|
| H | H | H | $OCH_3$ | $CR^6$ | N | $-NH_2$ |
| H | H | H | $OCH_3$ | $CR^6$ | N | -NHMe |
| H | H | H | $OCH_3$ | $CR^6$ | N | -NHEt |
| H | H | H | $OCH_3$ | $CR^6$ | N | -NHPh |
| H | H | H | $OCH_3$ | $CR^6$ | N | $-NH-CH_2Ph$ |
| H | H | H | $OCH_3$ | $CR^6$ | N | -NH- 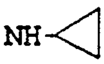 |
| H | H | H | $OCH_3$ | $CR^6$ | N | -NH-i-Pr |
| H | H | H | $OCH_3$ | $CR^6$ | N | -NH- 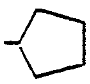 |
| H | H | H | $OCH_3$ | $CR^6$ | N | -NH- 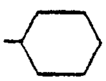 |
| H | H | H | $OCH_3$ | $CR^6$ | N | $-NMe_2$ |
| H | H | H | $OCH_3$ | $CR^6$ | N | $-NEt_2$ |
| H | H | H | $OCH_3$ | $CR^6$ | N | $-N(iPr)_2$ |
| H | H | H | $OCH_3$ | $CR^6$ | N | - 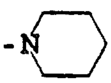 |
| H | H | H | $OCH_3$ | $CR^6$ | N | - 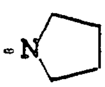 |
| H | H | H | $OCH_3$ | $CR^6$ | N | - 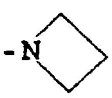 |
| H | H | H | $OCH_3$ | $CR^6$ | N | - 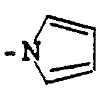 |
| H | H | H | $OCH_3$ | $CR^6$ | N | - 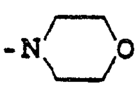 |
| H | H | H | $OCH_3$ | $CR^6$ | N | - 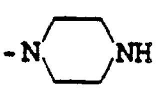 |

| $R^1$ | $R^2$ | $R^3$ | $R^6$ | X | Y | Z |
|---|---|---|---|---|---|---|
| H | H | H | $OCH_3$ | N | $CR^6$ | $-NH_2$ |
| H | H | H | $OCH_3$ | N | $CR^6$ | -NHMe |
| H | H | H | $OCH_3$ | N | $CR^6$ | -NHEt |
| H | H | H | $OCH_3$ | N | $CR^6$ | -NHPh |
| H | H | H | $OCH_3$ | N | $CR^6$ | $-NH-CH_2Ph$ |
| H | H | H | $OCH_3$ | N | $CR^6$ | 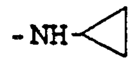 |
| H | H | H | $OCH_3$ | N | $CR^6$ | -NH-i-Pr |
| H | H | H | $OCH_3$ | N | $CR^6$ | 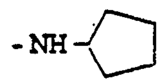 |
| H | H | H | $OCH_3$ | N | $CR^6$ | 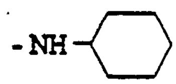 |
| H | H | H | $OCH_3$ | N | $CR^6$ | $-NMe_2$ |
| H | H | H | $OCH_3$ | N | $CR^6$ | $-NEt_2$ |
| H | H | H | $OCH_3$ | N | $CR^6$ | $-N(iPr)_2$ |
| H | H | H | $OCH_3$ | N | $CR^6$ | 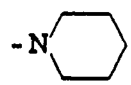 |
| H | H | H | $OCH_3$ | N | $CR^6$ | 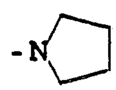 |
| H | H | H | $OCH_3$ | N | $CR^6$ | 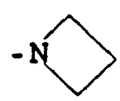 |
| H | H | H | $OCH_3$ | N | $CR^6$ | 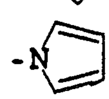 |
| H | H | H | $OCH_3$ | N | $CR^6$ | 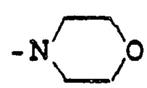 |
| H | H | H | $OCH_3$ | N | $CR^6$ | 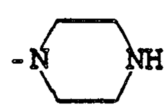 |

Die neuen Verbindungen der Formel I und ihre Salze besitzen wertvolle pharmakologische Eigenschaften.

Sie hemmen deutlich die Magensäuresekretion und weisen darüber hinaus eine ausgezeichnete Magen- und Darmschutzwirkung auf.

Unter "Magen- und Darmschutz" wird in diesem Zusammenhang die Verhütung und Behandlung gastrointestinaler Krankheiten, insbesondere gastrointestinaler entzündlicher Krankheiten und Läsionen (wie z.B. Ulcus ventriculi, Ulcus duodeni, Gastritis, hyperazider oder medikamentös bedingter Reizmagen) verstanden, die beispielsweise durch Mikroorganismen, Bakterientoxine, Medikamente (z.B. Antiphlogistika und Antirheumatika), Chemikalien (z.B. Ethanol), Magensäure oder Streßsituationen verursacht werden können.

Aufgrund ihrer ausgezeichneten Eigenschaften sind die substituierten Thienoimidazole der Formel I und ihre pharmakologisch verträglichen Salze für den Einsatz in der Human- und Veterinärmedizin hervorragend geeignet, wobei sie insbesondere zur Behandlung und Prophylaxe von Krankheiten des Magens und Darms und solcher Krankheiten, die auf einer überhöhten Magensäuresekretion beruhen, verwendet werden.

Es wurde gefunden, daß auch die Colon-$H^+$/$K^+$-ATPase (vgl. Gustin, Goodman, J. Biol. Chem. 256 - [1981] 10651-10656) in vitro stark durch Verbindungen gehemmt wird, die beim Behandeln der erfindungsgemäßen Verbindungen der Formel I mit Säure (z.B. mit Natriumacetat/HCl-Puffer mit einem pH-Wert von etwa 4-5,5) entstehen. Solche Umwandlungsprodukte können sich auch in vivo bei der Passage der Verbindungen der Formel I durch den Magen-Darm-Trakt bilden. In welchem Umfang sie gebildet werden, hängt vom Substitutionsmuster und vom pH ab.

Der Colon-$H^+$/$K^+$-ATPase wird ein entscheidender Einfluß auf das Elektrolytgleichgewicht an der Darmschleimhaut zugeschrieben. Colon-$H^+$-$K^+$ -ATPase-Hemmer, wie die oben genannten, können daher in dieses Gleichgewicht eingreifen und zu Behandlung von Krankheiten mit gestörtem Elektrolytgleichgewicht dienen.

Die Erfindung betrifft daher auch die Verwendung von Verbindungen der Formel I bzw. deren Säure-Umwandlungsprodukte bei der Behandlung von Durchfallerkrankungen. Beispiele solcher Krankheiten sind entzündliche Darmerkrankungen, wie Cholera, Paratyphus, Reisediarrhoe oder andere Formen der sekretorischen Diarrhoe aber auch andere Darmerkrankungen wie Colitis ulcerosa und regionale Enteritis.

Die Erfindung betrifft daher weiter die erfindungsgemäßen Verbindungen der Formel I zur Anwendung bei der Behandlung und Prophylaxe der vorstehend genannten Krankheiten.

Ebenso umfaßt die Erfindung die Verwendung der erfindungsgemäßen Verbindungen bei der Herstellung von Arzneimitteln, die zur Behandlung und Prophylaxe der vorstehend genannten Krankheiten eingesetzt werden.

Ein weiterer Gegenstand der Erfindung sind Arzneimittel, die ein oder mehrere Verbindungen der allgemeinen Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoffe) entweder als solche, oder vorzugsweise in Kombination mit geeigneten pharmazeutischen Hilfsstoffen in Form von Tabletten, Dragees, Kapseln, Suppositorien, Emulsionen, Suspensionen oder Lösungen eingesetzt, wobei der Wirkstoffgehalt vorteilhafterweise zwischen 0,1 und 96 % beträgt.

Welche Hilfsstoffe für die gewünschte Arzneimittelformulierungen geeignet sind, ist dem Fachmann aufgrund seines Wissens geläufig. Neben Lösemitteln, Gelbildern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Die Wirkstoffe können oral oder parenteral appliziert werden, wobei die orale Applikation bevorzugt ist.

Im allgemeinen hat es sich in der Humanmedizin als vorteilhaft erwiesen, den oder die Wirkstoffe bei oraler Gabe in einer Tagesdosis von etwa 0,01 bis etwa 20 mg/kg Körpergewicht, gegebenenfalls in Form mehrerer, vorzugsweise 1 bis 4 Einzelgaben zur Erzielung des gewünschten Ergebnisses zu verabreichen. Bei der parenteralen Applikation können ähnliche bzw. (insbesondere bei der intravenösen Verabreichung der Wirkstoffe) in der Regel niedrigere Dosierungen zur Anwendung kommen. Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder ihre Salze zur Behandlung der obengenannten Krankheiten eingesetzt werden, so können die pharmazeutischen Zubereitungen auch einen oder mehrere pharmakologisch aktive Bestandteile anderer Arzneimittelgruppen, wie Antibiotika, Antacida, beispielsweise Aluminiumhydroxid, Magnesiumaluminat, Sucralfat, Bi-Salze; Tranquilizer, wie Benzodiazepine, beispielsweise Diazepam; Spasmolytika, wie z.B. Bietamiverin, Camylofin; Anticholinergica, wie z.B. Pirenzepin, Telenzepin, Oxyphencylimin, Phencarbamid; Lokalanaesthetika, wie z.B. Tetracain, Procain; gegebenenfalls auch Gastrinantagonisten, Fermente, Vitamine oder Aminosäuren enthalten.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen.

Als inerte Träger können z.B. Gummi arabicum, Magnesia, Magnesiumcarbonat, Milchzucker, Glucose oder Stärke, insbesondere Maisstärke verwendet werden. Dabei kann die Zubereitung als Trocken- oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel für die neuen aktiven Verbindungen und die entsprechenden physiologisch verträglichen Salze kommen z.B. in Frage: Wasser, physiologische Kochsalzlösungen oder Alkohole, z.B. Ethanol, Propanol oder Glycerin, daneben auch Zuckerlösungen wie Glucose- oder Mannitlösungen, oder auch eine Mischung aus den verschiedenen genannten Lösungsmitteln.

Die nachstehenden Beispiele dienen zur Erläuterung der Erfindung, ohne daß diese darauf beschränkt wäre.

**Beispiel 1**

2-[4-Morpholino-2-pyrimidinylmethylsulfinyl]-1H-thieno[3,4-d]imidazol

a) Eine Mischung aus 120 g Methoxyacetamidinhydrochlorid und 300 g Formylessigsäureethylester-Natriumsalz in 1,5 l Wasser wurde bei Raumtemperatur 3 Tage lang gerührt. Nach dem Einengen der Reaktionslösung wurde mit Salzsäure angesäuert und mit Dichlormethan extrahiert. Nach säulenchromatographischer Reinigung wurden 100 g 2-Methoxymethyl-4-pyrimidon erhalten. (Schmp. 126° C)

b) Eine Suspension aus 100 g 2-Methoxymethyl-4-pyrimidon und 500 ml Phosphoroxychlorid wurde langsam bis zur Bildung einer klaren Lösung erhitzt. Überschüssiger Phosphoroxychlorid wurde destilliert, der Rückstand mit Eiswasser hydrolysiert und mit Natronlauge auf pH 10 eingestellt. Nach der Extraktion mit Dichlormethan und säulenchromatographischer Reinigung wurden 101 g 2-Methoxymethyl-4-Chloropyrimidin als Öl erhalten.

c) Eine Lösung aus 12 g 2-Methoxymethyl-4-Chloropyrimidin und 15 ml Morpholin in 50 ml Dichlormethan wurde 3 Stunden bei Raumtemperatur gerührt. Die Reaktionslösung wurde mehrmals mit Wasser gewaschen. Die organische Phase wurde getrocknet und ergab nach säulenchromatographischer Reinigung 13 g 2-Methoxymethyl-4-morpholinopyrimidin als Öl.

d) Eine Lösung aus 13 g 2-Methoxymethyl-4-morpholinopyrimidin in 100 ml Dichlormethan wurde unter Stickstoffatmosphäre bei -25° C tropfenweise mit einer Lösung aus 24 ml Bortribromid in 100 ml Dichlormethan versetzt. Nach 30 Minuten bei -25° C wurde die Lösung weitere 2 Stunden bei 0° C stehen gelassen und dann auf Eis gegeben. Mit Natronlauge wurde die Lösung basisch eingestellt und mit Dichlormethan extrahiert. Nach dem Trocknen ($Na_2SO_4$) und Einengen wurde mit Diethylether versetzt und 5,5 g 2-Hydroxymethyl-4-morpholinopyrimidin als Feststoff erhalten. (Schmp. 76-77° C)

e) Eine Lösung aus 5,5 g 2-Hydroxymethyl-4-morpholinopyrimidin und 17 ml Thionylchlorid in 200 ml Dichlormethan wurde 3 Stunden lang bei Raumtemperatur gerührt. Nach dem Einengen wurde mit Diethylether versetzt und das ausgefallene 2-Chloromethyl-4-morpholinopyrimidin-hydrochlorid abfiltriert. (Ausbeute 6,0 g; Schmp. 215° C)

f) Eine Mischung aus 1 g Natriummethylat, 1,3 g 2-Mercaptothieno[3,4-d]imidazol und 2 g 2-Chlormethyl-4-morpholinopyrimidin-hydrochlorid in 50 ml Methanol wurde 2 Stunden lang unter Rückfluß erhitzt. Anschließend wurde das Gemisch auf Eiswasser gegeben und mit Dichlormethan extrahiert. Nach säulenchromatographischer Reinigung wurden 1,6 g 2-(4-Morpholinopyrimidin-2-yl-methylthio)thieno[3,4-d]imidazol als Feststoff erhalten. (Schmp. 185° C [Zers. ])

g) Zu einer Suspension aus 300 mg 2-(4-Morpholinopyrimidin-2-yl-methylthio)-1H-thieno[3,4-d]-imidazol in einer Mischung von 30 ml Dichlormethan und 30 ml Natriumhydrogencarbonatlösung wurde bei 0° C portionsweise 200 mg m-Chlorperbenzoesäure zugegeben. Nach 30 Minuten schied sich zwischen den Phasen 2-(4-Morpholinopyrimidin-2-yl-methylsulfinyl)-1H-thieno[3,4-d]imidazol-Na-Salz ab, das abfiltriert und mit Dichlormethan gewaschen wurde. (Ausbeute: 300 mg; Schmp. 165° C [Zers. ])

h) 300 mg 2-(4-Morpholinopyrimidin-2-yl-methylsulfinyl)-1H-thieno[3,4-d]imidazol-Na-Salz wurde in einer Mischung von 10 ml Dichlormethan und 10 ml Wasser suspendiert und mit gesättigter Ammoniumchloridlösung auf pH 6 eingestellt. Die wäßrige Phase wurde mit Dichlormethan extrahiert, die organische Phase wurde getrocknet ($Na_2SO_4$) und eingeengt. Nach radialer Schichtchromatographie wurden 200 mg 2-(4-Morpholinopyrimidin-2-yl-methylsulfinyl)-1H-thieno[3,4-d]imidazol als Feststoff erhalten. (Schmp. 164° C-[Zers. ])

Die folgenden Verbindungen wurden in analoger Weise erhalten:

**Beispiel 2**

2-[4-Piperidino-2-pyrimidinylmethylthio]-1H-thieno[3,4-d]imidazol, F: 197° C [Zers. ]

**Beispiel 3**

2-[4-(N,N-Dimethylamino)-2-pyrimidinylmethylthio]-1H-thieno[3,4-d]imidazol, F: 193° C [Zers. ]

**Beispiel 4**

2-[5-Methyl-4-morpholino-2-pyrimidinylmethylthio]-1H-thieno[3,4-d]imidazol, F: 177° C [Zers. ]

**Beispiel 5**

2-[5-Methyl-4-piperidino-2-pyrimidinylmethylthio]-1H-thieno[3,4-d]imidazol, F: 117° C [Zers. ]

**Beispiel 6**

2-[5-Methyl-4-piperazino-2-pyrimidinylmethylthio]-1H-thieno[3,4-d]imidazol, Schmp. 230° C [Zers. ]

**Beispiel 7**

2-[5-Methyl-4-(4-methylphenylamino)pyrimidin-2-yl-methylthio]-1H-thieno[3,4-d]imidazol, Schmp. 77° C [Zers. ]

**Beispiel 8**

2-[5-Methyl-4-(4-methylphenylamino)pyrimidin-2-yl-methylsulfinyl]-1H-thieno[3,4-d]imidazol, Schmp. 115° C [Zers. ]

**Beispiel 9**

2-(5-Chlor-4-piperidinopyrimidin-2-yl-methylthio)-1H-thieno[3,4-d]imidazol, Schmp. 132° C [Zers. ]

**Beispiel 10**

2-(5-Chlor-4-piperidinopyrimidin-2-yl-methylsulfinyl)-1H-thieno[3,4-d]imidazol, Schmp. 59° C [Zers. ]

**Beispiel 11**

2-(5-Chlor-4-morpholinopyrimidin-2 yl-methylthio)-1H-thieno[3,4-d]imidazol, Schmp. 165° C [Zers. ]

**Beispiel 12**

2-(5-Chlor-4-morpholinopyrimidin-2-yl-methylsulfinyl)-1H-thieno[3,4-d]imidazol, Schmp. 81° C [Zers. ]

**Beispiel 13**

2-(5-Chlor-4-morpholinopyrimidin-2-yl-methylsulfonyl)-1H-thieno[3,4-d]imidazol, Schmp. 155° C [Zers. ]

**Beispiel 14**

2-[5-Methyl-4-(4-methylpiperazin-1-yl)pyrimidin-2-yl-methylthio]-1H-thieno[3,4-d]imidazol, Schmp. 159° C [Zers. ])

**Beispiel 15**

2-(5-Chlor-4-pyrrolidinopyrimidin-2-yl-methylthio)-1H-thieno[3,4-d]imidazol, Schmp. 188° C [Zers. ]

**Beispiel 16**

2-(5-Chlor-4-pyrrolidinopyrimidin-2-yl-methylsulfinyl)-1H-thieno[3,4-d]imidazol, Schmp. 86° C [Zers. ]

**Beispiel 17**

2-[5-Chlor-4-(N,N-dimethylamino)-pyrimidin-2-yl-methylsulfinyl]-1H-thieno[3,4-d]imidazol, Schmp. 178° C [Zers. ]

**Beispiel 18**

2-[5-Chlor-4-(N,N-dimethylamino)-pyrimidin-2-yl-methylsulfinyl]-1H-thieno[3,4-d]imidazol, Schmp. 112°C [Zers. ]

**Beispiel 19**

2-(4-Benzylpiperidino-5-Chlor-pyrimidin-2-yl-methylsulfonyl)-1H-thieno[3,4-d]imidazol, Schmp. 166°C [Zers. ]

**Beispiel 20**

2-(4-Methoxypyrimidin-2-yl-methylsulfinyl)-1H-thieno[3,4-d]imidazol

a) 650 g Formylessigsäureethylester-Natriumsalz wurden in 4 l kaltem Wasser gelöst und mit 450 g AcetamidinHydrochlorid versetzt. Nach zweitägigem Stehen bei Raumtemperatur wurde die Lösung im Vakuum bis zur Trockne eingedampft und der Rückstand mit heißem Ethanol extrahiert. Nach dem Einengen der ethanolischen Extrakte und säulenchromatographischer Reinigung wurden 240 g 2-Methylpyrimid-4-on erhalten. (Schmp. 212°C)

b) 11 g 2-Methylpyrimid-4-on wurden in 50 ml Phosphoroxychlorid eingetragen und das Gemisch auf 80°C erhitzt, bis eine klare Lösung entstand. Überschüssiges Phosphoroxychlorid wurde im Vakuum abdestilliert und der Rückstand in Eiswasser gegeben. Unter Kühlung wurde die Lösung mit Kalilauge neutralisiert und mit Dichlormethan extrahiert. Die organischen Extrakte wurden getrocknet ($Na_2SO_4$), im Vakuum eingeengt, und nach der Säulenchromatographie wurden 8 g 4-Chlor-2-methylpyrimidin erhalten. (Schmp. 50-60°C)

c) Zu einer Lösung aus 1,2 g Natrium in 50 ml absolutem Methanol wurden 6,5 g 4-Chlor-2-methylpyrimidin gegeben. Das Reaktionsgemisch wurde 1 Stunde lang unter Rückfluß erhitzt, dann im Vakuum eingeengt, mit Wasser versetzt und mit Diethylether extrahiert. Nach der Säulenchromatographie wurden 6 g 4-Methoxy-2-methylpyrimidin als Öl erhalten.

d) Eine Suspension aus 6 g 4-Methoxy-2-methylpyrimidin, 4 g Trichlorisocyanursäure in 50 ml Tetrachlorkohlenstoff wurde unter Rückfluß erhitzt. Nach Beendigung der Reaktion wurde mit Wasser versetzt, auf pH 8 bis 9 eingestellt und mit Dichlormethan extrahiert. Nach der Säulenchromatographie wurden 4 g 2-Chlormethyl-4-methoxypyrimidin als Öl erhalten.

e) Zu einer Lösung aus 3,1 g 2-Mercaptothieno[3,4-d]imidazol in 100 ml absolutem Dimethylformamid wurden bei Raumtemperatur 900 mg Natriumhydrid-Suspension (55 %ig) und nach 15 Minuten 3,3 g 2-Chlormethyl-4-methoxypyrimidin gegeben. Anschließend wurde 1 Stunde bei Raumtemperatur gerührt, das Reaktionsgemisch auf Eis gegeben und mit Dichlormethan extrahiert. Nach dem Trocknen ($Na_2SO_4$) und Einengen der organischen Phase wurde über eine Kieselgelsäule gereinigt und 1,6 g 2-(4-Methoxypyrimidin-2-yl-methylthio)-1H-thieno[3,4-d]imidazol erhalten. (Schmp. 165°C [Zers. ])

f) 1,1 g 2-(4-Methoxypyrimidin-2-yl-methylthio)-1H-thieno[3,4-d]imidazol wurden in 100 ml Dichlormethan gelöst und bei -10°C portionsweise mit 820 mg m-Chlorperbenzoesäure versetzt. Anschließend wurde 15 Minuten bei Raumtemperatur gerührt, mit Natriumhydrogencarbonatlösung ausgeschüttelt, die organische Phase über Natriumsulfat getrocknet und eingeengt. Nach chromatographischer Reinigung wurden 300 mg 2-(4-Methoxypyrimidin-2-yl-methylsulfinyl)-1H-thieno[3,4 d]imidazol erhalten. (Schmp. 125°C [Zers. ])

In Analogie zu Beispiel 20 wurden die folgenden Verbindungen erhalten:

**Beispiel 21**

2-(4-Ethoxypyrimidin-2-yl-methylthio)-1H-thieno[3,4-d]imidazol, Schmp. 164°C [Zers. ]

**Beispiel 22**

2-(4-Ethoxypyrimidin-2-yl-methylsulfinyl)-1H-thieno[3,4-d]imidazol, Schmp. 115° C [Zers. ]

**Beispiel 23**

2-(4-Isopropoxypyrimidin-2-yl-methylthio)-1H-thieno[3,4 d]imidazol, Schmp. 182° C [Zers. ]

**Beispiel 24**

2-(4-Isopropoxypyrimidin-2-yl-methylsulfinyl)-1H-thieno[3,4-d]imidazol, Schmp. 118° C [Zers. ]

**Beispiel 25**

2-(6-Morpholinopyrimidin-4-yl-methylsulfinyl)-1H-thieno[3,4-d]-imidazol

a) Eine Lösung aus 250 g 4-Methoxyacetessigsäuremethylester und 200 g Formamidinacetat in 1,5 l Methanol wurde mit einer Lösung aus 86 g Natrium in 1,5 l Methanol versetzt. Die Reaktionslösung wurde 48 Stunden lang auf 60° C erwärmt, dann im Vakuum eingeengt und in Eiswasser gegeben. Nach der Neutralisation mit Salzsäure wurde mit Dichlormethan extrahiert, über Natriumsulfat getrocknet und im Vakuum eingeengt. Durch Reinigung über eine Kieselgelsäule wurden 100 g 6-Hydroxy-4-methoxymethylpyrimidin erhalten (Schmp. 160° C)

b) 50 g 6-Hydroxy-4-methoxymethylpyrimidin wurden in 375 ml Phosphoroxychlorid so lange erhitzt, bis eine klare Lösung entstanden war. Das überschüssige Phosphoroxychlorid wurde im Vakuum abdestilliert und der Rückstand in Eiswasser gegeben. Nach der Extraktion mit Dichlormethan, dem Trocknen über Natriumsulfat wurde die Lösung im Vakuum eingeengt. Es wurden 58 g 6-Chlor-4-methoxymethylpyrimidin als Öl erhalten, das ohne weitere Reinigung in der nächsten Stufe eingesetzt wurde.

c) 29 g 6-Chlor-4-methoxymethylpyrimidin wurden in 200 ml Tetrahydrofuran gelöst und mit 32 g Morpholin versetzt. Nach 3 Stunden bei Raumtemperatur wurde abgesaugt und das Filtrat im Vakuum eingeengt.
Es wurden 37 g 6-Morpholino-4-methoxymethylpyrimidin erhalten, (Schmp. 56° C).

d) Zu einer Lösung aus 5,3 g 6-Morpholino-4-methoxymethylpyrimidin in 100 ml Dichlormethan wurde bei 0° C unter Stickstoffatmosphäre 38 ml 1 m Bortribromidlösung in $CH_2Cl_2$ getropft. Die Reaktionslösung wurde in Eiswasser gegeben und mit Natronlauge auf pH 13 eingestellt. Nach der Extraktion mit Dichlormethan wurde die organische Phase über Natriumsulfat getrocknet und im Vakuum eingeengt.
Es wurden 3 g 4-Hydroxymethyl-6-morpholinopyrimidin erhalten (Schmp. 138° C)

e) Zu einer Lösung aus 3 g 4-Hydroxymethyl-6-morpholinpyrimidin in 30 ml Dichlormethan wurde bei 0° C eine Lösung aus 36 ml Thionylchlorid in 30 ml Dichlormethan getropft. Die Reaktionslösung wurde auf Raumtemperatur kommen gelassen und im Vakuum eingeengt. Nach Zugabe von Diethylether fielen 3,2 g 4-Chlormethyl-6-morpholinopyrimidin-Hydrochlorid aus (Schmp. 139° C [Zers. ])

f) 1 g 4-Chlormethyl-6-morpholinopyrimidin-Hydrochlorid wurden mit 625 mg 2-Mercaptothieno[3,4-d]imidazol analog umgesetzt, wie in Beispiel 1f beschrieben.
Es wurden 420 mg 2-(6-Morpholinopyrimidin-4-yl-methylthio)-1H-thieno[3,4-d]imidazol erhalten (Schmp. 178° C [Zers. ])

g) 180 mg 2-(6-Morpholinopyrimidin-4-yl-methylthio)thieno-1H-[3,4-d]imidazol wurden mit m-Chlorperbenzoesäure analog zu Beispiel 1g oxidiert.
Es wurden 140 mg 2-(6-Morpholinopyrimidin-4-yl-methylsulfinyl)-1H-thieno[3,4-d]imidazol erhalten (Schmp. 107° C [Zers. ])

Die folgenden Verbindungen wurden in analoger Weise erhalten:

**Beispiel 26**

2-(6-Piperidinopyrimidin-4-yl-methylthio)-1H-thieno[3,4-d]imidazol, Schmp. 161 °C [Zers. ]

**Beispiel 27**

2-(5-Chlor-6-piperidinopyrimidin-4-yl-methylthio)-1H-thieno[3,4-d]imidazol, Schmp. 152 °C [Zers. ]

**Beispiel 28**

2-(5-Chlor-6-piperidinopyrimidin-4-yl-methylsulfinyl)-1H-thieno[3,4-d]imidazol, Schmp. 89 °C [Zers. ]

**Beispiel 29**

2-(5-Chlor-6-morpholinopyrimidin-4-yl-methylthio)-1H-thieno[3,4-d]imidazol, Schmp. 72 °C [Zers. ]

**Beispiel 30**

2-(5-Chlor-6-morpholinopyrimidin-4-yl-methylsulfinyl)-1H-thieno-[3,4-d]imidazol, Schmp. 90 °C [Zers. ]

**Beispiel 31**

2-(5-Methoxy-4-piperidinopyrimidin-2-yl-methylsulfinyl)-1Hthieno[3,4-d]imidazol

a) Ersetzt man wie in Beispiel 1a beschrieben das Formylessigsäureethylester-Natriumsalz durch 2-Methoxy-3-oxo-propionsäureethylester-Natriumsalz, so erhält man entsprechend 5-Methoxy-2-methoxymethyl-pyrimidin-4-on, Schmp. 61 °C
Setzt man das 5-Methoxy-2-methoxymethyl-pyrimidin-4-on weiter um wie im Beispiel 1b-g beschrieben, so erhält man über die Zwischenstufen 31b-f die Endstufe 31g

b) 4-Chlor-5-methoxy-2-methoxymethyl-pyrimidin, Öl

c) 5-Methoxy-2-methoxymethyl-4-piperidino-pyrimidin, Öl

d) 2-Hydroxy-5-methoxy-4-piperidino-pyrimidin, Schmp. 102 °C

e) 2-Chlormethyl-5-methoxy-4-piperidino-pyrimidin-Hydrochlorid, Schmp. 189 °C [Zers. ]

f) 2-(5-Methoxy-2-piperidinopyrimidin-2-yl-methylthio)-1H-thieno[3,4-d]imidazol, Schmp. 103 °C [Zers. ]

g) 2-(5-Methoxy-4-piperidinopyrimidin-2-yl-methylsulfinyl)-1H-thieno[3,4-d]imidazol, Schmp. 155 °C [Zers. ]

**Beispiel 32**

2-(5-Methoxy-4-morpholinopyrimidin-2-yl-methylsulfinyl)-1H-thieno[3,4-d]imidazol, Schmp. 130 °C [Zers. ]

**Beispiel 33**

2-(5-Benzyloxy-4-morpholinopyrimidin-2-yl-methylsulfinyl)-1H-thieno[3,4-d]imidazol, Schmp. 56° C [Zers. ]

**Beispiel 34**

2-[5-Benzyloxy-4-(4-Benzylpiperidino)pyrimidin-2-yl-methylsulfinyl]-1H-thieno[3,4-d]imidazol, Schmp. 62° C [Zers. ]

**Beispiel 35**

2-(5-Benzyloxy-4-piperidinopyrimidin-2-yl-methylsulfinyl)-1H-thieno[3,4-d]imidazol, Schmp. 72° C [Zers. ]

**Beispiel 36**

2-[5-Benzyloxy-4-(N,N-dimethylamino)pyrimidin-2-yl-methylsulfinyl]-1H-thieno[3,4-d]imidazol, Schmp. 65° C [Zers. ]

**Beispiel 37**

2-[5-(4-Fluorbenzyloxy)-4-(4-benzylpiperidino)pyrimidin-2-yl-methylsulfiny]-1H-thieno[3,4-d]imidazol, Schmp. 119° C [Zers. ]

**Beispiel 38**

2-[5-(4-Fluorbenzyloxy)-4-piperidinopyrimidin-2-yl-methylsulfinyl]-1H-thieno[3,4-d]imidazol, Schmp. 110° C [Zers. ]

**Beispiel 39**

2-[5-(4-Fluorbenzyloxy)-4-morpholinopyrimidin-2-yl-methylsulfinyl]-1H-thieno[3,4-d]imidazol, Schmp. 125° C [Zers. ]

**Beispiel 40**

2-[4-Piperidino-5-(2,2,2-trifluorethoxy)-pyrimidin-2-yl-methylsulfinyl]-1H-thieno[3,4-d]imidazol, Schmp. 168° C [Zers. ]

**Beispiel 41**

2-[4-(N,N-Dimethylamino)-5-(2,2,2-trifluorethoxy)pyrimidin-2-yl-methylsulfinyl]-1H-thieno[3,4-d]imidazol, Schmp. 155 °C [Zers. ]

**Beispiel 42**

2-[4-Morpholino-5-(2,2,2-trifluorethoxy)pyrimidin-2-yl-methylsulfinyl]-1H-thieno[3,4-d]imidazol, Schmp. 165 °C [Zers. ]

**Beispiel 43**

2-[4-Pyrrolo-5-(2,2,2-trifluorethoxy)pyrimidin-2-yl-methylsulfinyl]-1H-thieno[3,4-d]imidazol, Schmp. 155 °C [Zers. ]

**Beispiel 44**

2-[4-Piperidino-5-(4,4,4,3,3,2,2-heptafluorbutoxy)pyrimidin-2-yl-methylsulfinyl]-1H-thieno[3,4-d]imidazol, Schmp. 172 °C [Zers. ]

**Ansprüche**

1. Verbindung der Formel I

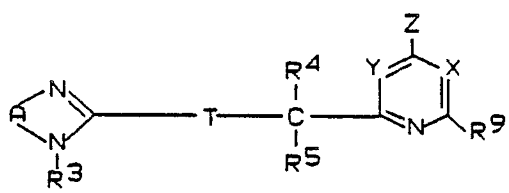

(I)

in welcher

A für a) 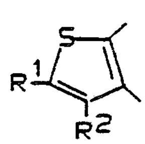 , b) 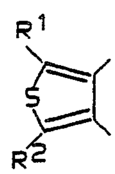 oder c) 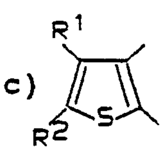 steht,

T -S-, -SO- oder $-SO_2-$ bedeutet,
$R^1$ und $R^2$ gleich oder verschieden sind und Wasserstoff, Halogen, Cyano, Nitro, Trifluormethyl, $(C_1-C_6)$-Alkyl, $(C_1-C_6)$-Hydroxyalkyl, $(C_1-C_6)$-Alkoxy, $(C_1-C_4)$-Fluoralkoxy, $-OCF_2Cl$, $-O-CF_2-CHFCl$, $(C_1-C_6)$-Alkylmercapto, $(C_1-C_6)$-Alkylsulfinyl, $(C_1-C_6)$-Alkylsulfonyl, $(C_1-C_6)$-Alkylcarbonyl, $(C_1-C_6)$-Alkoxycarbonyl, Carbamoyl, N-$(C_1-C_4)$-Alkylcarbamoyl, N,N-Di-$(C_1-C_4)$-alkylcarbamoyl, $(C_1-C_6)$-Alkylcarbonyloxy, $(C_3-C_8)$-Cycloalkyl, Phenyl, Benzyl, Phenoxy, Benzyloxy, Anilino, N-Methylanilino, Phenylmercapto, Phenylsulfonyl, Phenylsulfinyl, Sulfamoyl, N-$(C_1-C_4)$-Alkylsulfamoyl oder N,N-Di-$(C_1-C_4)$-alkylsulfamoyl bedeuten, oder, falls A wie oben unter (a) oder (c) definiert ist, auch gemeinsam $-[CH_2]_n-$ oder -CH=CH-CH=CH- bedeuten können, wobei eine $CH_2$-Gruppe gegebenenfalls durch O, S, SO oder $SO_2$ ersetzt ist,
$R^3$ Wasserstoff, Alkanoyl, $(C_1-C_6)$-Alkylcarbamoyl oder eine andere physiologisch verträgliche, vorzugsweise im sauren Medium und/oder unter physiologischen Bedingungen abspaltbare $N^{im}$-Schutz-

gruppe bedeutet,
$R^4$ und $R^5$ gleich oder verschieden sind und Wasserstoff oder $(C_1-C_3)$-Alkyl bedeuten,
X Stickstoff bedeutet und Y $CR^6$ bedeutet, oder
X $CR^6$ bedeutet und Y Stickstoff bedeutet,
$R^6$ Wasserstoff, Halogen, $(C_1-C_6)$-Alkyl, Trifluormethyl, $(C_1-C_6)$-Alkoxy, $-O-C_pH_{(2p+1-q)}F_q$, $(C_1-C_6)$-Alkoxy-$(C_1-C_6)$-alkyl, $(C_6-C_{12})$-Aryl, $(C_6-C_{12})$-Aryl-$(C_1-C_6)$-alkoxy, $(C_1-C_9)$-Heteroaryl, $(C_6-C_{12})$-Aryloxy, wobei der entsprechende Arylteil gegebenenfalls durch Halogen oder Trifluormethyl substituiert sein kann, Cyano, Nitro, $(C_1-C_6)$-Alkylamino, Di-$(C_1-C_6)$-alkylamino oder Amino bedeutet,
Z $NR^7R^8$, $OR^{10}$ oder $SR^{10}$ bedeutet
$R^7$ und $R^8$ gleich oder verschieden sind und Wasserstoff, $(C_1-C_6)$-Alkyl, $(C_6-C_{12})$-Aryl, $(C_7-C_{13})$-Aralkyl oder $(C_3-C_6)$-Cycloalkyl bedeuten oder zusammen mit dem Stickstoff, an den die gebunden sind, für Azetidino, Pyrrolidino, Piperidino, Piperazino, Morpholino oder N-$(C_1-C_4)$-Alkylpiperazino stehen, die gegebenenfalls durch eine oder zwei gleiche oder verschiedene $(C_1-C_6)$-Alkylgruppen substituiert sind,
$R^9$ Wasserstoff, Halogen, $(C_1-C_3)$-Alkyl, $(C_1-C_4)$-Alkoxy, Benzyloxy oder $(C_1-C_7)$-Alkoxy-$(C_1-C_3)$-alkyl bedeutet,
$R^{10}$ Wasserstoff, $(C_1-C_4)$-Alkyl, $(C_7-C_{13})$-Aralkyl, $(C_6-C_{12})$-Aryl, Vinyl, Allyl oder einen substituierten Alkylrest der Formel $C_p\ H_{(2p+1-q)}F_q$ bedeutet,
n = 3 oder 4 ist,
p = 1, 2, 3 oder 4 ist und
q = 1 bis 2 p + I ist,
sowie deren physiologisch verträgliche Salze.

2. Verbindung der Formel I gemäß Anspruch 1, in welcher $R^9$ Wasserstoff bedeutet oder deren physiologisch verträgliche Salze.

3. Verbindung der Formel I gemäß Anspruch 1 oder 2, in welcher A wie im Anspruch 1 unter (b) definiert ist oder deren physiologisch verträgliche Salze.

4. Verbindung der Formel I gemäß einem oder mehreren der Ansprüche 1 bis 3, in welcher T für -SO- steht oder deren physiologisch verträgliche Salze.

5. Verbindung der Formel I gemäß einem oder mehreren der Ansprüche 1 bis 4, in welcher
$R^1$ und $R^2$ gleich oder verschieden sind und Wasserstoff, $(C_1-C_3)$-Alkyl, Halogen, $(C_1-C_4)$-Alkoxy, oder $(C_1-C_4)$-Alkoxycarbonyl bedeuten,
$R^3$ wie im Anspruch 1 definiert ist,
$R^4$ und $R^5$ jeweils Wasserstoff bedeuten und
$R^6$ Amino, Wasserstoff, Halogen, $(C_1-C_6)$-Alkyl, $(C_1-C_6)$-Alkoxy, $-O-Cp(H_{2p+1-q})F_q$, $(C_6-C_{12})$-Aryloxy, $(C_6-C_{12})$-Aryl-$(C_1-C_6)$-alkoxy, wobei der entsprechende Arylteil gegebenenfalls durch Halogen oder Trifluormethyl substituiert sein kann, Cyano, Nitro, $(C_1-C_6)$-Alkylamino, Di-$(C_1-C_6)$-alkylamino oder $(C_1-C_6)$-Alkoxy-$(C_1-C_3)$-alkyl bedeutet;
$R^7$ und $R^8$ gleich oder verschieden und Wasserstoff, $(C_1-C_6)$-Alkyl, $(C_3-C_6)$-Cycloalkyl oder Phenyl bedeuten, oder diese zusammen mit dem Stickstoff, an den sie gebunden sind, für Azetidino, Pyrrolidino, Piperidino, Piperazino, Morpholino oder N-$(C_1-C_4)$Alkylpiperazino stehen, und
$R^9$ und $R^{10}$ wie im Anspruch 1 definiert sind
oder deren physiologisch verträgliche Salze.

6. Verbindung der Formel I gemäß einem oder mehreren der Ansprüche 1 bis 5, in welcher
$R^1$ und $R^2$ gleich oder verschieden sind und Wasserstoff oder $(C_1-C_3)$-Alkyl bedeuten,
$R^3$ Wasserstoff bedeutet,
$R^4$ und $R^5$ jeweils Wasserstoff bedeuten, $R^6$ Wasserstoff, Amino, $(C_1-C_6)$-Alkoxy, Trifluorethoxy, Heptafluorbutoxy, Benzyloxy, Fluorbenzyloxy, Difluorbenzyloxy,Trifluorbenzyloxy, Chlor, Nitro, Cyano, Methyl oder Ethyl bedeutet,
$R^9$ Wasserstoff bedeutet,
$R^7$ und $R^8$ gleich oder verschieden sind und Wasserstoff, Phenyl oder $(C_1-C_6)$-Alkyl bedeuten, oder diese zusammen mit dem Stickstoff, an den sie gebunden sind, für Azetidino, Piperidino, Pyrrolidino, Morpholino, Piperazino oder N-$(C_1-C_4)$-Alkylpiperazino stehen, und
$R^{10}$ $(C_1-C_4)$-Alkyl, Phenyl oder Benzyl bedeutet
oder deren physiologisch verträglichen Salze.

7. Verfahren zur Herstellung einer Verbindung der Formel I gemäß einem oder mehreren der Ansprüche 1 bis 6, dadurch gekennzeichnet, daß man
a) eine Verbindung der Formel II

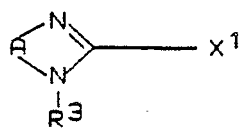

(II)

in welcher A, $R^1$, $R^2$ und $R^3$ wie im Anspruch 1 definiert sind und

$X^1$ i. eine Abgangsgruppe oder

ii. -SH, $-S^-M^+$ oder $-SO_2^-\ M^+$ bedeutet, wobei $M^+$ für ein Kation steht

umsetzt mit einer Verbindung der Formel III

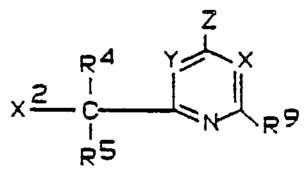

(III)

in welcher X, Y, Z, $R^4$, $R^5$, $R^6$, $R^7$, $R^8$, $R^9$ und $R^{10}$ wie im Anspruch 1 definiert sind und

$X^2$ im oben genannten Fall i. -SH, $-S^-M^+$ oder $-SO_2^-M^+$, wobei $M^+$ für ein Kation steht, und im oben genannten Fall ii.eine Abgangsgruppe bedeutet, oder

b) eine Verbindung der Formel IV,

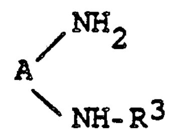

(IV)

in welcher A, $R^1$, $R^2$ und $R^3$ wie oben definiert sind, umsetzt mit einer Verbindung der Formel V

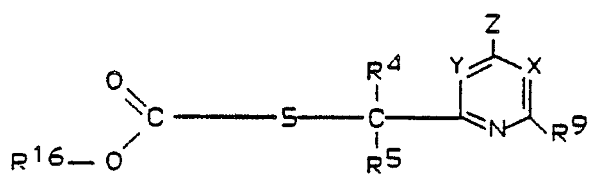

(V)

in welcher X, Y, Z, $R^4$, $R^5$, $R^6$, $R^7$, $R^8$, $R^9$ und $R^{10}$ wie oben definiert sind und $R^{16}$ für eine veresternde Gruppe steht,

i. in einer Verbindung der Formel I (eine) gegebenenfalls vorhandene -S-Gruppe(n) gewünschtenfalls zu(r) -SO oder $-SO_2$-Gruppe(n) oxidiert,

ii. in einer Verbindung der Formel I (eine) gegebenenfalls vorhandene -SO-Gruppe(n) gewünschtenfalls zu(r) $-SO_2$-Gruppe(n) oxidiert,

iii. eine Verbindung der Formel I, worin $R^3$ für Wasserstoff steht, gewünschtenfalls acyliert, alkyliert oder aralkyliert,

iv. eine Verbindung der Formel I, worin $R^3$ nicht Wasserstoff bedeutet, gewünschtenfalls verseift und

v. eine Verbindung der Formel I gewünschtenfalls in ihr physiologisch verträgliches Salz überführt,

wobei zwei oder mehr der Maßnahmen i.-iv. auch in einer anderen als der angegebenen Reihenfolge ausgeführt werden können.

8. Verwendung einer Verbindung gemäß einem oder mehreren der Ansprüche 1 bis 6 als Heilmittel.

9. Verwendung einer Verbindung gemäß einem oder mehreren der Ansprüche 1 bis 6 als Magensäuresekretionshemmer.

10. Mittel enthaltend eine Verbindung gemäß einem oder mehreren der Ansprüche 1 bis 6.

11. Verfahren zur Herstellung eines Mittels gemäß Anspruch 10, dadurch gekennzeichnet, daß man eine Verbindung gemäß einem oder mehreren der Ansprüche 1 bis 6 in eine geeignete Darreichungsform bringt.

Patentansprüche fur folgende Vertragsstaaten: ES, GR

1. Verfahren zur Herstellung einer Verbindung der Formel I

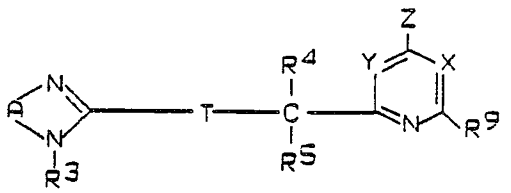
(I)

in welcher

A für a) 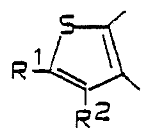 , b) 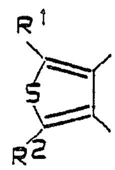 oder c) 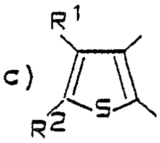 steht,

T -S-, -SO- oder $-SO_2-$ bedeutet,
$R^1$ und $R^2$ gleich oder verschieden sind und Wasserstoff, Halogen, Cyano, Nitro, Trifluormethyl, $(C_1-C_6)$-Alkyl, $(C_1-C_6)$-Hydroxyalkyl, $(C_1-C_6)$-Alkoxy, $(C_1-C_4)$-Fluoralkoxy, $-OCF_2Cl$, $-O-CF_2-CHFCl$, $(C_1-C_6)$-Alkylmercapto, $(C_1-C_6)$-Alkylsulfinyl, $(C_1-C_6)$-Alkylsulfonyl, $(C_1-C_6)$-Alkylcarbonyl, $(C_1-C_6)$-Alkoxycarbonyl, Carbamoyl, N-$(C_1-C_4)$-Alkylcarbamoyl, N,N-Di-$(C_1-C_4)$-alkylcarbamoyl, $(C_1-C_6)$-Alkylcarbonyloxy, $(C_3-C_8)$-Cycloalkyl, Phenyl, Benzyl, Phenoxy, Benzyloxy, Anilino, N-Methylanilino, Phenylmercapto, Phenylsulfonyl, Phenylsulfinyl, Sulfamoyl, N-$(C_1-C_4)$-Alkylsulfamoyl oder N,N-Di-$(C_1-C_4)$-alkylsulfamoyl bedeuten, oder, falls A wie oben unter (a) oder (c) definiert ist, auch gemeinsam $-[CH_2]_n-$ oder -CH=CH-CH=CH- bedeuten können, wobei eine $CH_2$-Gruppe gegebenenfalls durch O, S, SO oder $SO_2$ ersetzt ist,
$R^3$ Wasserstoff, Alkanoyl, $(C_1-C_6)$-Alkylcarbamoyl oder eine andere physiologisch verträgliche, vorzugsweise im sauren Medium und/oder unter physiologischen Bedingungen abspaltbare $N^{im}$-Schutzgruppe bedeutet,
$R^4$ und $R^5$ gleich oder verschieden sind und Wasserstoff oder $(C_1-C_3)$-Alkyl bedeuten,
X Stickstoff bedeutet und Y $CR^6$ bedeutet, oder
X $CR^6$ bedeutet und Y Stickstoff bedeutet,
$R^6$ Wasserstoff, Halogen, $(C_1-C_6)$-Alkyl, Trifluormethyl, $(C_1-C_6)$-Alkoxy, $-O-C_pH_{(2p+1-q)}F_q$, $(C_1-C_6)$-Alkoxy-$(C_1-C_6)$-alkyl, $(C_6-C_{12})$-Aryl, $(C_6-C_{12}$-Aryl-$(C_1-C_6)$-alkoxy, $(C_1-C_9)$-Heteroaryl, $(C_6-C_{12})$-Aryloxy, wobei der entsprechende Arylteil gegebenenfalls durch Halogen oder Trifluormethyl substituiert sein kann, Cyano, Nitro, $(C_1-C_6)$-Alkylamino, Di-$(C_1-C_6)$-alkylamino oder Amino bedeutet,
Z $NR^7R^8$, $OR^{10}$ oder $SR^{10}$ bedeutet,
$R^7$ und $R^8$ gleich oder verschieden sind und Wasserstoff, $(C_1-C_6)$-Alkyl, $(C_6-C_{12})$-Aryl, $(C_7-C_{13})$-Aralkyl oder $(C_3-C_6)$-Cycloalkyl bedeuten oder zusammen mit dem Stickstoff, an den die gebunden sind, für Azetidino, Pyrrolidino, Piperidino, Piperazino, Morpholino oder N-$(C_1-C_4)$-Alkylpiperazino stehen, die gegebenenfalls durch eine oder zwei gleiche oder verschiedene $(C_1-C_6)$-Alkylgruppen substituiert sind,
$R^9$ Wasserstoff, Halogen, $(C_1-C_3)$-Alkyl, $(C_1-C_4)$-Alkoxy, Benzyloxy oder $(C_1-C_7)$-Alkoxy-$(C_1-C_3)$-alkyl bedeutet,
$R^{10}$ Wasserstoff, $(C_1-C_4)$-Alkyl, $(C_7-C_{13})$ Aralkyl, $(C_6-C_{12})$-Aryl, Vinyl, Allyl oder einen substituierten Alkylrest der Formel $C_pH_{(2p+1-q)}F_q$ bedeutet,
n = 3 oder 4 ist,
p = 1, 2, 3 oder 4 ist und
q = 1 bis 2 p + 1 ist,
oder deren physiologisch verträglichen Salzen,
dadurch gekennzeichnet, daß man
a) eine Verbindung der Formel II

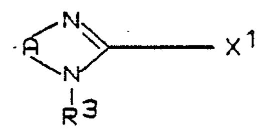

(II)

in welcher A, $R^1$, $R^2$ und $R^3$ wie oben definiert sind und

$X^1$ i. eine Abgangsgruppe oder

ii. -SH, $-S^-M^+$ oder-$SO_2^-M^+$ bedeutet, wobei $M^+$ für ein Kation steht,

umsetzt mit einer Verbindung der Formel III

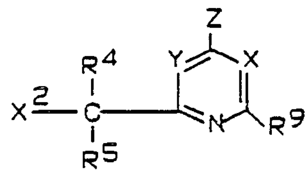

(III)

in welcher X, Y, Z, $R^4$, $R^5$, $R^6$, $R^7$, $R^8$, $R^9$ und $R^{10}$ wie oben definiert sind und

$X^2$ im oben genannten Fall i. -SH, $-S^-M^+$ oder $-SO_2^-M^+$, wobei $M^+$ für ein Kation steht, und im oben genannten Fall ii. eine Abgangsgruppe bedeutet, oder

b) eine Verbindung der Formel IV,

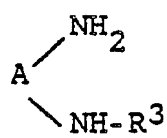

(IV)

in welcher A, $R^1$, $R^2$ und $R^3$ wie oben definiert sind, umsetzt mit einer Verbindung der Formel V

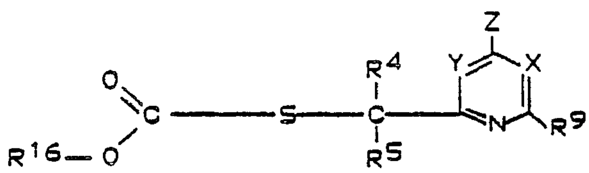

(V)

in welcher X, Y, Z, $R^4$, $R^5$, $R^6$, $R^7$, $R^8$, $R^9$ und $R^{10}$ wie oben definiert sind und $R^{16}$ für eine veresternde Gruppe steht,

i. in einer Verbindung der Formel I (eine) gegebenenfalls vorhandene -S-Gruppe(n) gewünschtenfalls zu(r) -SO oder -$SO_2$-Gruppe(n) oxidiert,

ii. in einer Verbindung der Formel I (eine) gegebenenfalls vorhandene -SO-Gruppe(n) gewünschtenfalls zu(r)-SO oder -$SO_2$-Gruppe(n) oxidiert,

iii. eine Verbindung der Formel I, worin $R^3$ für Wasserstoff steht, gewünschtenfalls acyliert, alkyliert oder aralkyliert,

iv. eine Verbindung der Formel I, worin $R^3$ nicht Wasserstoff bedeutet, gewünschtenfalls verseift und

v. eine Verbindung der Formel I gewünschtenfalls in ihr physiologisch verträgliches Salz überführt, wobei zwei oder mehr der Maßnahmen i.-iv. auch in einer anderen als der angegebenen Reihenfolge ausgeführt werden können.

2. Verfahren gemäß Anspruch 1, dadurch gekennzeichnet, daß eine Verbindung der Formel I hergestellt wird, in welcher $R^9$ Wasserstoff bedeutet oder deren physiologisch verträgliche Salze.

3. Verfahren gemäß Anspruch 1 oder 2, dadurch gekennzeichnet, daß eine Verbindung der Formel I hergestellt wird, in welcher A wie im Anspruch 1 unter (b) definiert ist oder deren physiologisch verträgliche Salze.

4. Verfahren gemäß einem oder mehreren der Ansprüche 1 bis 3, dadurch gekennzeichnet, daß eine Verbindung der Formel I hergestellt wird, in welcher T für -SO- steht oder deren physiologisch verträgliche Salze.

5. Verfahren gemäß einem oder mehreren der Ansprüche 1 bis 4, dadurch gekennzeichnet, daß eine Verbindung der Formel I hergestellt wird, in welcher

$R^1$ und $R^2$ gleich oder verschieden sind und Wasserstoff, $(C_1-C_3)$-Alkyl, Halogen, $(C_1-C_4)$-Alkoxy, oder $(C_1-C_4)$-Alkoxycarbonyl bedeuten,

$R^3$ wie im Anspruch 1 definiert ist,

$R^4$ und $R^5$ jeweils Wasserstoff bedeuten und

$R^6$ Amino, Wasserstoff, Halogen, $(C_1-C_6)$-Alkyl, $(C_1-C_6)$-Alkoxy, -O-$C_p(H_{2p+1-q})F_q$, $(C_6-C_{12})$-Aryloxy, $(C_6-C_{12})$-Aryl-$(C_1-C_6)$-alkoxy, wobei der entsprechende Arylteil gegebenenfalls durch Halogen oder Trifluormethyl substituiert sein kann, Cyano, Nitro, $(C_1-C_6)$-Alkylamino, Di-$(C_1-C_6)$-alkylamino oder $(C_1-C_6)$-Alkoxy-$(C_1-C_3)$-alkyl bedeutet;

$R^7$ und $R^8$ gleich oder verschieden und Wasserstoff, $(C_1-C_6)$-Alkyl, $(C_3-C_6)$-Cycloalkyl oder Phenyl bedeuten, oder diese zusammen mit dem Stickstoff, an den sie gebunden sind, für Azetidino, Pyrrolidino, Piperidino, Piperazino, Morpholino oder N-$(C_1-C_4)$-Alkylpiperazino stehen, und

$R^9$ und $R^{10}$ wie im Anspruch 1 definiert sind

oder deren physiologisch verträgliche Salze.

6. Verfahren gemäß einem oder mehreren der Ansprüche 1 bis 5, dadurch gekennzeichnet, daß eine Verbindung der Formel I hergestellt wird, in welcher

$R^1$ und $R^2$ gleich oder verschieden sind und Wasserstoff oder $(C_1-C_3)$-Alkyl bedeuten,

$R^3$ Wasserstoff bedeutet,

$R^4$ und $R^5$ jeweils Wasserstoff bedeuten,

$R^6$ Wasserstoff, Amino, $(C_1-C_6)$-Alkoxy, Trifluorethoxy, Heptafluorbutoxy, Benzyloxy, Fluorbenzyloxy, Difluorbenzyloxy, Trifluorbenzyloxy, Chlor, Nitro, Cyano, Methyl oder Ethyl bedeutet,

$R^9$ Wasserstoff bedeutet,

$R^7$ und $R^8$ gleich oder verschieden sind und Wasserstoff, Phenyl oder $(C_1-C_6)$-Alkyl bedeuten, oder diese zusammen mit dem Stickstoff, an den sie gebunden sind, für Azetidino, Piperidino, Pyrrolidino, Morpholino, Piperazino oder N-$(C_1-C_4)$-Alkylpiperazino stehen, und

$R^{10}$ $(C_1-C_4)$-Alkyl, Phenyl oder Benzyl bedeutet

oder deren physiologisch verträgliche Salze.

7. Verwendung einer Verbindung gemäß einem oder mehreren der Ansprüche 1 bis 6 als Heilmittel.

8. Verfahren zur Herstellung eines Mittels enthaltend eine Verbindung hergestellt gemäß einem oder mehrerer der Ansprüche 1 bis 6, dadurch gekennzeichnet, daß man eine Verbindung der Formel I in eine geeignete Darreichungsform bringt.